# EUROPEAN PATENT SPECIFICATION

(11) **EP 0 996 726 B1**
(45) Date of publication and mention of the grant of the patent: **10.12.2003**
(21) Application number: 98945293.3
(22) Date of filing: 28.08.1998
(51) Int. Cl.: C12N 15/12, C07K 14/705, C12N 15/85, C12N 5/10, C07K 16/28, A61K 38/17, A61K 48/00, A61K 39/395, G01N 33/68

(54) **T-CELL MEMBRANE PROTEIN (TIRC7), PEPTIDES AND ANTIBODIES DERIVED THEREFROM AND USES THEREOF**
T-ZELL-MEMBRANPROTEIN (TIRC7), DAVON ABGELEITETE PEPTIDE UND ANTIKÖRPER UND IHRE VERWENDUNGEN
PROTEINE MEMBRANAIRE DES LYMPHOCYTES T (TIRC7), PEPTIDES ET ANTICORPS DERIVES DE CETTE PROTEINE ET LEURS UTILISATIONS

(30) Priority: 29.08.1997 DE 19738710; 12.02.1998 DE 29802653 U
(43) Date of publication of application: 03.05.2000
(60) Divisional application: 03015210.2
(73) Proprietor: BRIGHAM AND WOMEN'S HOSPITAL, Boston, Massachusetts 02115 (US); Utku, Nalan, 10719 Berlin (DE)
(72) Inventor: UTKU, Nalan, D-10719 Berlin (DE); GULLANS, Steven, R., Natick, MA 02115 (US); MILFORD, Edgar, L., Dover, MA 02030 (US)
(74) Representative: Steinecke, Peter, Dr.
(86) International application number: EP9805462
(87) International publication number: WO99011782

(56) References cited:
- YI-PING LI ET AL.: "Molecular cloning and characterization of a putative novel human osteoclast-specific 116-kDa vacuolar proton pump subunit" BIOCHEMICAL AND BIOPHYSICAL RESEARCH COMMUNICATIONS, vol. 218, no. 3, 26 January 1996, pages 813-821, XP002087029 ORLANDO, FL US cited in the application
- CHONG-KIL LEE ET AL.: "Cloning of a cDNA for a T cell produced molecule with a putative immune regulatory role" MOLECULAR IMMUNOLOGY, vol. 27, no. 11, November 1990, pages 1137-1144, XP002087030 cited in the application

## Description

### FIELD OF THE INVENTION

The present invention pertains generally to a T cell immune response cDNA 7 (TIRC7) encoding a novel T-cell transmembrane protein as well as peptides und polypeptides derived therefrom and antibodies recognizing said (poly)peptides. In a first aspect, the present invention relates to TIRC7 cDNA and its encoded protein. In a further aspect, the present invention relates to polynucleotides derived from said TIRC7 cDNA encoding a peptide or polypeptide being capable of inhibiting T-cell stimulation through the T-cell membrane protein (TIRC7). Furthermore, the present invention relates to vectors comprising such polynucleotides and host cells transformed therewith as well as their use in the production of the above-defined peptides or polypeptides. In addition, the present invention relates to the (poly)peptide encoded by said polynucleotides or obtainable by the method of the invention. In another important aspect the present invention relates to antibodies against said peptides and polypeptides that are capable of inhibiting T-cell stimulation through the T-cell membrane protein (TIRC7). The present invention additionally relates to pharmaceutical and diagnostic compositions comprising the aformentioned peptide, polypeptide, or antibody. Furthermore, the present invention relates to methods and uses for modulating immune responses through the novel TIRC7 membrane protein as well as to pharmaceutical compositions comprising agents which act on the TIRC7 membrane protein or its ligand. Also, the invention relates to the use of the before-described polynucleotide, vector, peptide, polypeptide, or antibody for the preparation of pharmaceutical compositions for use in organ transplantation, for the treatment of autoimmune, allergic or infectious diseases, or for treatment of tumors. Furthermore, the present invention relates to methods for modulating (antigen-specific) T cell unresponsiveness. The present invention encompasses methods for inducing, maintaining or reversing T cell unresponsiveness by inhibiting or stimulating an (unresponsive) T cell through the novel TIRC7 membrane protein.

Several documents are cited throughout the text of this specification. Each of the documents cited herein (including any manufacturer's specifications, instructions, etc.) are hereby incorporated herein by reference; however, there is no admission that any document cited is indeed prior art as to the present invention.

### BACKGROUND OF THE INVENTION

T cell activation is a serial process involving multiple signaling pathways and sequential changes in gene expression resulting in differentiation of T cells into distinct subpopulations, i.e. Th1 and Th2, which are distinguishable by their pattern of cytokine production and characterize the mode of the cellular immune response (Abbas et al., 1996; Crabtree, 1989). The T cell response is initiated by the interaction of the antigen-specific T cell receptor (TCR) with peptide presented by major histocompatibility complex (MHC) molecules on the surface of antigen presenting cells (APCs). Additional signals are provided by a network of receptor-ligand interactions mediated by a number of membrane proteins such as CD28/CTLA4 and B7, CD40/CD40L, LFA-1 and ICAM-1 (Lenschow et al., 1996; Linsley and Ledbetter, 1993; Xu et al., 1994, Bachmann et al., 1997; Schwartz, 1992), collectively called costimulatory signals (Perez et al., 1997). These membrane proteins can alter T cell activation in distinct ways (Bachmann et al., 1997) and regulate the immune response by the integration of positive and negative signals provided by these molecules (Bluestone, 1995; Perez et al., 1997). Many of the agents which are effective in modulating the cellular immune response either interfere with the T cell receptor (Cosimi et al., 1981), block costimulatory signaling (Larsen et al., 1996; Blazar et al., 1996; Kirk et al. 1997; Linsley et al., 1992; Turka et al., 1992) or inhibit intracellular activation signals downstream from these primary cell membrane triggers (Schreiber and Crabtree, 1992). Therapeutic prevention of T cell activation in organ transplantation and autoimmune diseases presently relies on panimmunosuppressive drugs interfering with downstream intracellullar events. Specific modulation of the T cell response remains a longstanding goal in immunological research.

### SUMMARY OF THE INVENTION

The present invention relates to polynucleotides encoding a novel T-cell membrane protein. Furthermore, the present invention relates to peptides and polypeptides derived therefrom as well as to antibodies capable of inhibiting T-cell stimulation through the novel T-cell membrane protein. More particulary, the present invention relates to applications in the medical field that directly arise from the polynucleotides. peptides, (poly)peptides and antibodies of the invention. Additionally, the present invention relates to a novel method for testing activators and inhibitors of T-cell proliferation. The pharmaceutical compositions, methods and uses of the invention are useful therapeutically in situations where it is desirable to modulate (antigen-specific) immune responses, e.g., inducing and maintain (antigen-specific) T-cell unresponsiveness or restore (antigen-specific) T-cell responsiveness. For example, it may be necessary to induce or maintain T-cell unresponsiveness in a subject who has received an organ or bone marrow transplant to prevent graft rejection by inhibiting stimulation through the TIRC7 membrane protein. In addition, T-cell unresponsiveness can be maintained by blocking TIRC7 stimulation in a subject who has an autoimmune disease to alleviate symptoms of the autoimmune disease. In these cases, a TIRC7 inhibitory agent is administered to the subject in an amount and over a period of time sufficient to maintain T-cell unresponsiveness. Alternatively, T-cell unresponsiveness can be reversed in a subject bearing a tumor to stimulate a tumor specific T-cell response or in a subject receiving a vaccine to enhance the efficacy of the vaccine. For example, a cell (e.g., a tumor cell) can be modified to express a TIRC7 ligand or a TIRC7 stimulatory agent can be administered to the subject bearing a tumor or who has had a tumor surgically removed to prevent recurrence of the tumor. Additionally, antigen-specific responsiveness can be restored to anergized T-cells *in vitro* by stimulating the T-cells through TIRC7. Responsive T-cells generated *in vitro* can then be administered to a subject.

### DETAILED DESCRIPTION OF THE PRESENT INVENTION

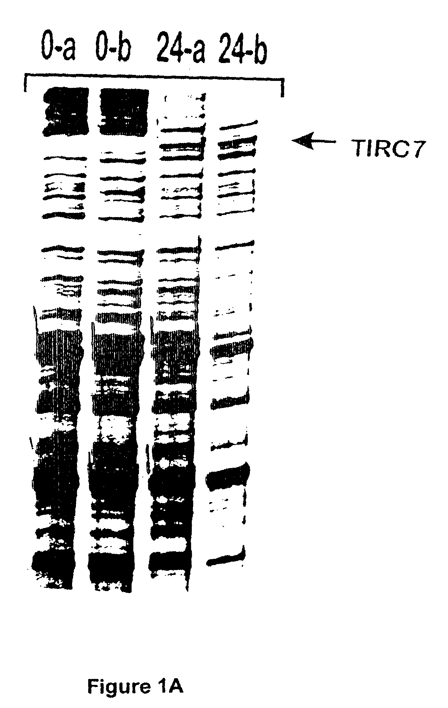

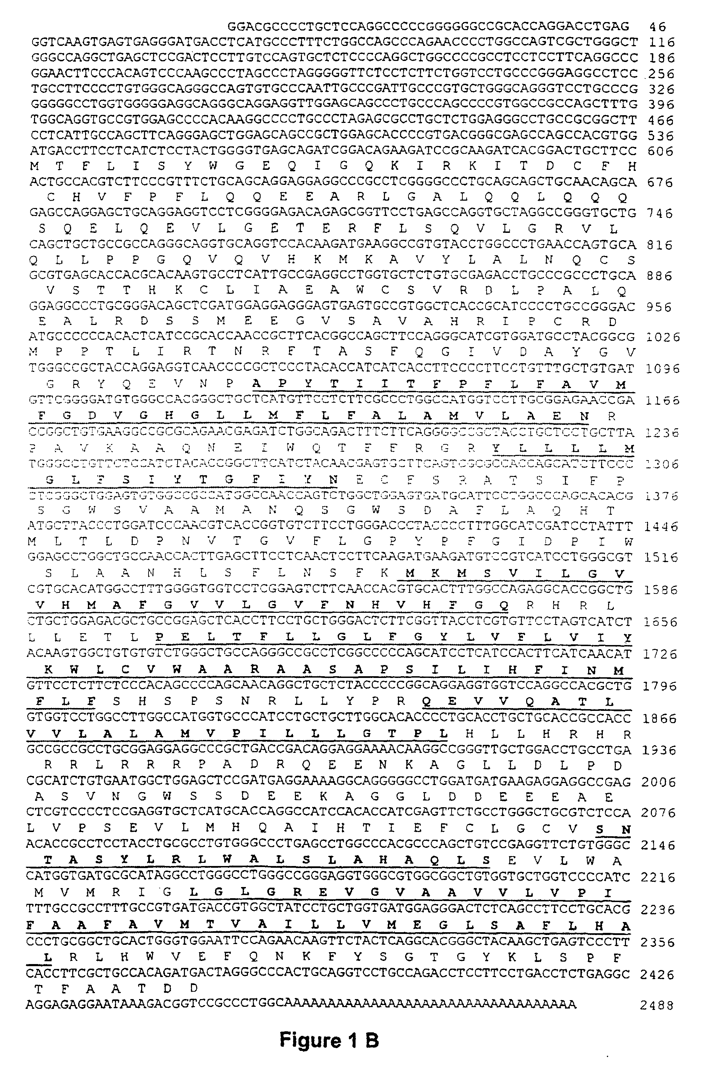

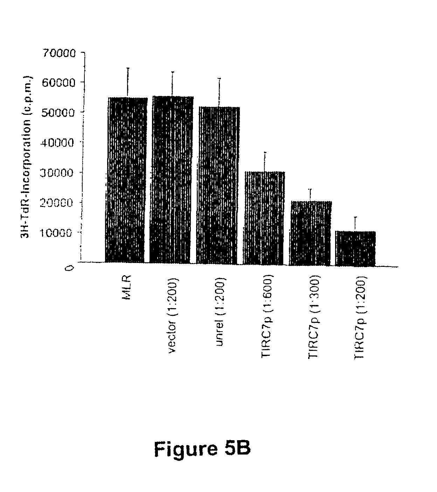

In view of the need of therapeutic means for the treatment of diseases related to immune responses of the human body, the technical problem of the invention is to provide means and methods for the modulation of T-cell responses which are particularly useful in organ transplantation and autoimmune diseases.
The solution to this technical problem is achieved by providing the embodiments characterized in the claims, namely a novel T-cell membrane protein encoded by T-cell Immune Response cDNA7 (TIRC7) is described which exhibits a central role in T-cell activation *in vitro* and *in vivo*. TIRC7 mRNA is transiently upregulated in the early phase of T-cell activation via a calcineurin-dependent pathway.
In a first set of experiments, the TIRC7 protein encoding cDNA has been cloned and characterized; see the first part of Example 1 and Figure 1. Furthermore, said cDNA was subjected to in vitro translation and preliminary results obtained in MLR inhibition tests with the in vitro translated TIRC7 protein suggested a putative immunosuppressive potential of said protein, see Example 2 with Figure 5B.
A second (independent) set of experiments performed in accordance with the present invention (Examples 2 to 4), surprisingly revealed that modulation of TIRC7 membrane protein mediated signals with specific anti-TIRC7 membrane protein antibodies *in vitro* efficiently prevents T-cell proliferation and IL-2 secretion which is reversible by exogenous IL-2. Anti-TIRC7 membrane protein antibodies specifically inhibit Th1 subset specific cytokine expression but spare the Th2 cytokines. Administration of said antibodies in rats significantly prolongs kidney allograft survival. The latter described results obtained in accordance with the present invention provide evidence for an essential role of TIRC7 membrane protein in the early events of T cell activation. Thus, targeting of TIRC7 membrane protein and its encoding gene provides a novel therapeutic approach for modulation of the immune response.

Accordingly, the invention relates to a polynucleotide encoding a TIRC7 membrane protein or a biologically active fragment thereof comprising a DNA sequence selected from the group consisting of
(i) DNA sequences comprising a nucleotide sequence encoding the amino acid sequence depicted in SEQ ID NO. 2 or SEQ ID NO. 13 from amino acid position 1 to 614 or from amino acid position 1 to 601;
(ii) DNA sequences comprising the nucleotide sequence depicted in SEQ ID NO. 1 or SEQ ID NO. 12;
(iii) DNA sequences comprising a nucleotide sequence encoding a fragment or derivative of the protein encoded by the DNA sequence of (i) or (ii); and
(iv) DNA sequences the complementary strand of which hybridizes with and which are at least 70% identical to the polynucleotide as defined in any one of (i) to (iii).

The term "TIRC7 membrane protein" as used in accordance with the present invention, denotes a protein involved in the signal transduction of T-cell activation and/or proliferation and that, preferably in a soluble form is capable of inhibiting or suppressing T-cell proliferation in response to alloactivation in a mixed lymphocyte culture or in response to mitogens when exogeneously added to the culture. Studies which had been carried out within the scope of the present invention revealed that soluble *in vitro* translated TIRC7 protein is able to efficiently suppress in a dose dependent manner the proliferation of T-cells in response to alloactivation in a mixed lymphocyte culture or in response to mitogens; see Example 2, Figure 5B.

The term "biologically active fragment thereof" refers to peptides and polypeptides that are derived from said TIRC7 membrane protein and that are capable of inhibiting T-cell proliferation as defined above.

Previously, a cDNA fragment was amplified by the Reverse Transcription Differential Display Polymerase Chain Reaction (DDRT-PCR) technique from RNA from cells stimulated in mixed lymphocyte culture (MLC). The amino acid sequence of the 350 base-pair (bp) cDNA fragment thus obtained was reported to belong to a new human (H+)-ATPase proton pump homologue differentially expressed in alloactivated lymphocytes as the amino acid sequence had substantial homology to a rat and bovine vesicular (H+)-ATPase proton pump, respectively. While the function of the molecule was not known, it was also speculated that its homology to a mouse T-cell derived immunosuppressive protein (J6B7, Lee, Molecular Immunology 27 (1990), 1137-1144) suggested that the corresponding gene may be involved in immunomodulation. A full-length cDNA clone was isolated by means of the 350 base-pair cDNA fragment from a library of activated human T-cells. However, the amino acid sequence of the newly cloned cDNA did not reveal further insights into the putative function of the encoded protein although some initial experiments concerning the expression pattern of gene supposedly corresponding to the 350 bp fragment and its encoded, in vitro translated protein were reported and gave rise to speculation. Furthermore, the attempt to clone the mouse homologue with the help of either the 350 bp fragment or the full-length cDNA failed.

Further investigations revealed, that the amino acid sequence of the cDNA fragment identified by DDRT-PCR from RNA of stimulated T-lymphocytes corresponds to a transmembrane region. Thus, it could not be ruled out that due to conserved structures and amino acid sequences of such transmembrane regions the full-length cDNA clone did not actually correspond to the fragment identified by DDRT-PCR. For example, there are 5 amino acid differences in the amino acid sequence of the TIRC7 protein compared with that of the transmembrane region encoded by the 350 bp fragment. Furthermore, since DDRT-PCR analysis of RNA from human lymphocytes identified several genes whose expression was changed with alloactivation (multi) gene families may exist of similar structure and/or function while only one or few members of those genes are actually identified by the above-described technique. Hence, the relevance of the preliminary results described above and the actual biological function of this unknown protein being without precedent were absolutely unclear.
The preliminary results were all the more questioned in view of the recent publication of Lee, Biochem. Biophys. Res. Communications 218 (1996), 813-821, wherein the cloning and characterization of a human osteoclast-specific 116 kDa proton pump subunit was reported the cDNA of which comprises a nucleotide and an amino acid sequence which is substantially identical to those of the gene cloned from activated human T-cells. Thus, it appeared as if the previous results reflected an artefact and/or were obtained due to the activity of a pseudo-gene of the gene described in Lee, supra.

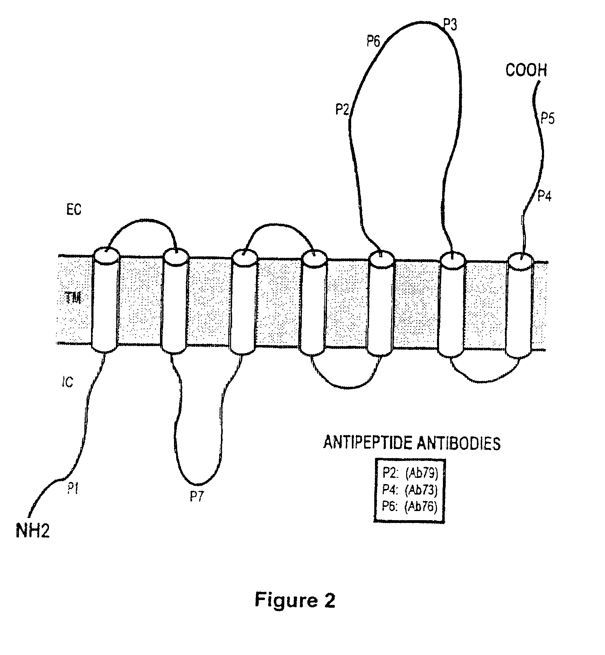

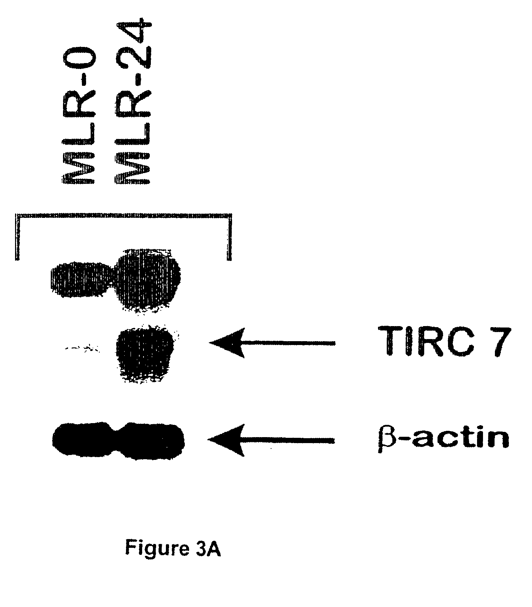

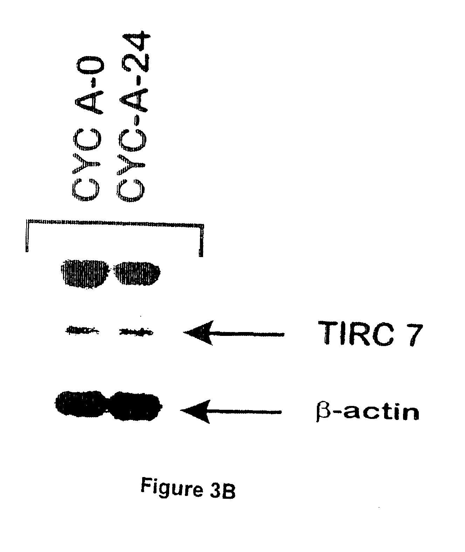

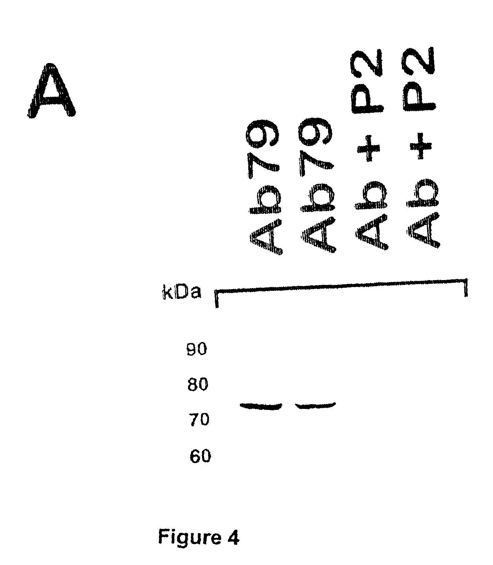

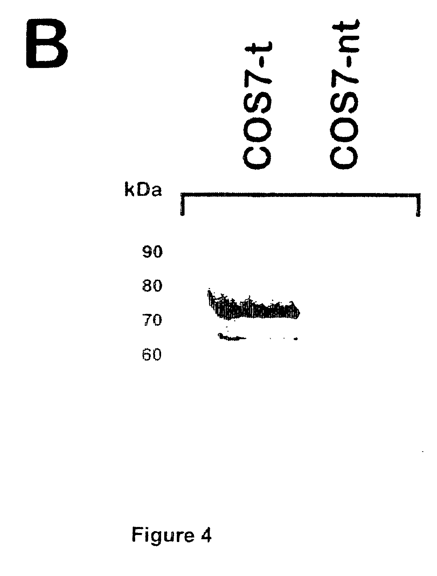

In accordance with the present invention a polynucleotide with the nucleotide sequence of the coding region as depicted in SEQ ID NO: 1 has been identified encoding a protein of 614 amino acids (SEQ ID NO: 2) with a molecular weight of 75 kDa. Experiments performed in accordance with the present invention revealed that TIRC7 membrane protein is expressed in all lymphoid tissues with low expression only in thymus, bone marrow and fetal liver and is transiently up regulated in lymphocytes after stimulation of the T-cell receptor, see Example 1, Figure 3. The TIRC7 encoding gene has been located by using the fluorescence-in situ-hybridization (FISH) method on the long arm of human chromosome 10 (13.4-13.5q) which is close to the breakpoint region of the bcl-gene associated with leukemia. The TIRC7 membrane protein is predominantly expressed on the cell membrane, consistent with a target for an external ligand; see Example 1, Figure 4. The seven transmembrane domain structure predicts three extracellular loops and an extracellularly oriented carboxy terminus; see Figure 2.

From the above it is evident that the nucleotide sequence depicted in SEQ ID NO. 1 encodes a novel class of T-cell membrane proteins. By the provision of this nucleotide sequence it is now possible to isolate identical or similar polynucleotides which code for proteins with the biological activity of TIRC7 from other species or organisms. Well-established approaches for the identification and isolation of such related sequences are, for example, the isolation from genomic or cDNA libraries using the complete or part of the disclosed sequence as a probe or the amplification of corresponding polynucleotides by polymerase chain reaction using specific primers. In accordance with the present invention, a further polynucleotide encoding a TIRC7 membrane protein was isolated using a nucleic acid molecule comprising the coding sequence of SEQ ID NO: 1 as a probe. The nucleotide sequence of said polynucleotide is given in SEQ ID NO: 12 encoding a protein having the amino acid sequence of SEQ ID NO: 13. The nucleotide and amino acid sequences of said TIRC7 membrane protein are substantially identical with those of the TIRC7 membrane protein encoded by SEQ ID NO: 1 except at amino acid position 121 (Arg → Gln) and, therefore, presumably represent allelic variants.

Thus, the invention also relates to polynucleotides which hybridize to the above described polynucleotides and differ at one or more positions in comparison to these as long as they encode a TIRC7 membrane protein as defined above. Such molecules comprise those which are changed, for example, by deletion(s), insertion(s), alteration(s) or any other modification known in the art in comparison to the above described polynucleotides either alone or in combination. Methods for introducing such modifications in the polynucleotides of the invention are well-known to the person skilled in the art; see, e.g., Sambrook et al. (Molecular cloning; A Laboratory Manual, Second Edition, Cold Spring Harbor Laboratory Press, Cold Spring Harbor NY (1989)). The invention also relates to polynucleotides the nucleotide sequence of which differs from the nucleotide sequence of any of the above-described polynucleotides due to the degeneracy of the genetic code.

With respect to the DNA sequences characterized under (iv) above, the term "hybridizing" in this context is understood as referring to conventional hybridization conditions, preferably such as hybridization in 50%formamide/6xSSC/0.1%SDS/100µg/ml ssDNA, in which temperatures for hybridization are above 37°C and temperatures for washing in 0.1xSSC/0.1%SDS are above 55°C. Most preferably, the term "hybridizing" refers to stringent hybridization conditions, for example such as described in Sambrook, supra.

Particularly preferred are polynucleotides which share 70%, preferably at least 85%, more preferably 90-95%, and most preferably 96-99% sequence identity with one of the above-mentioned polynucleotides and have the same biological activity. Such polynucleotides also comprise those which are altered, for example by nucleotide deletion(s), insertion(s), substitution(s), addition(s), and/or recombination(s) and/or any other modification(s) known in the art either alone or in combination in comparison to the above-described polynucleotides. Methods for introducing such modifications in the nucleotide sequence of the polynucleotide of the invention are well known to the person skilled in the art. Thus, the present invention encompasses any polynucleotide that can be derived from the above described polynucleotides by way of genetic engineering and that encode upon expression a TIRC7 membrane protein or a biologically active fragment thereof.

It is also immediately evident to the person skilled in the art that regulatory sequences may be added to the polynucleotide of the invention. For example, promoters, transcriptional enhancers and/or sequences which allow for induced expression of the polynucleotide of the invention may be employed. A suitable inducible system is for example tetracycline-regulated gene expression as described, e.g., by Gossen and Bujard (Proc. Natl. Acad. Sci. USA 89 (1992), 5547-5551) and Gossen et al. (Trends Biotech. 12 (1994), 58-62).

In a further embodiment, the invention relates to nucleic acid molecules of at least 15 nucleotides in length hybridizing specifically with a polynucleotide as described above or with a complementary strand thereof. Specific hybridization occurs preferably under stringent conditions and implies no or very little cross-hybridization with nucleotide sequences encoding no or substantially different proteins. Such nucleic acid molecules may be used as probes and/or for the control of gene expression. Nucleic acid probe technology is well known to those skilled in the art who will readily appreciate that such probes may vary in length. Preferred are nucleic acid probes of 17 to 35 nucleotides in length. Of course, it may also be appropriate to use nucleic acids of up to 100 and more nucleotides in length. The nucleic acid probes of the invention are useful for various applications. On the one hand, they may be used as PCR primers for amplification of polynucleotides according to the invention. Another application is the use as a hybridization probe to identify polynucleotides hybridizing to the polynucleotides of the invention by homology screening of genomic DNA libraries. Nucleic acid molecules according to this preferred embodiment of the invention which are complementary to a polynucleotide as described above may also be used for repression of expression of a gene comprising such a polynucleotide, for example due to an antisense or triple helix effect or for the construction of appropriate ribozymes (see, e.g., EP-B1 0 291 533, EP-A1 0 321 201, EP-A2 0 360 257) which specifically cleave the (pre)-mRNA of a gene comprising a polynucleotide of the invention. Selection of appropriate target sites and corresponding ribozymes can be done as described for example in Steinecke, Ribozymes, Methods in Cell Biology 50, Galbraith et al. eds Academic Press, Inc. (1995), 449-460. Standard methods relating to antisense technology have also been described (Melani, Cancer Res. 51 (1991), 2897-2901). Furthermore, the person skilled in the art is well aware that it is also possible to label such a nucleic acid probe with an appropriate marker for specific applications, such as for the detection of the presence of a polynucleotide of the invention in a sample derived from an organism.
The above described nucleic acid molecules may either be DNA or RNA or a hybrid thereof. Furthermore, said nucleic acid molecule may contain, for example, thioester bonds and/or nucleotide analogues, commonly used in oligonucleotide anti-sense approaches. Said modifications may be useful for the stabilization of the nucleic acid molecule against endo- and/or exonucleases in the cell. Said nucleic acid molecules may be transcribed by an appropriate vector containing a chimeric gene which allows for the transcription of said nucleic acid molecule in the cell. Such nucleic acid molecules may further contain ribozyme sequences as described above.

In this respect, it is also to be understood that the polynucleotide of the invention can be used for "gene targeting" and/or "gene replacement", for restoring a mutant gene or for creating a mutant gene via homologous recombination; see for example Mouellic, Proc. Natl. Acad. Sci. USA, 87 (1990), 4712-4716; Joyner, Gene Targeting, A Practical Approach, Oxford University Press.

In a preferred embodiment said nucleic acid molecules are labeled. Methods for the detection of nucleic acids are well known in the art, e.g., Southern and northern blotting, PCR or primer extension. In another preferred embodiment said nucleic acid molecules may be used for the suppression of TIRC7 expression.

The polynucleotide of the invention encoding the above described TIRC7 membrane protein or biologically active fragments thereof may be, e.g., DNA, cDNA, RNA or synthetically produced DNA or RNA or a recombinantly produced chimeric nucleic acid molecule comprising any of those polynucleotides either alone or in combination. Preferably said polynucleotide is part of a vector. Such vectors may comprise further genes such as marker genes which allow for the selection of said vector in a suitable host cell and under suitable conditions. Preferably, the polynucleotide of the invention is operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in prokaryotic host cells comprise, e.g., the P_{L}, lac, trp or tac promoter in E. coli, and examples for regulatory elements permitting expression in eukaryotic host cells are the AOX1 or GAL1 promoter in yeast or the CMV-, SV40- , RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the polynucleotide of the invention and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an C- or N-terminal identification peptide imparting desired characteristics, e.g., stabilization or simplified purification of expressed recombinant product. In this context, suitable expression vectors are known in the art such as Okayama-Berg cDNA expression vector pcDV1 (Pharmacia), pCDM8, pRc/CMV, pcDNA1, pcDNA3 (In-Vitrogene), or pSPORT1 (GIBCO BRL).
Preferably, the expression control sequences will be eukaryotic promoter systems in vectors capable of transforming or transfecting eukaryotic host cells, but control sequences for prokaryotic hosts may also be used. Once the vector has been incorporated into the appropriate host, the host is maintained under conditions suitable for high level expression of the nucleotide sequences, and, as desired, the collection and purification of the protein of the invention may follow; see, e.g., the appended examples.

In accordance with the above, the present invention relates to vectors, particularly plasmids, cosmids, viruses and bacteriophages used conventionally in genetic engineering that comprise a polynucleotide of the invention. Methods which are well known to those skilled in the art can be used to construct recombinant vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1989). Alternatively, the polynucleotides and vectors of the invention can be reconstituted into liposomes for delivery to target cells. The vectors containing the polynucleotides of the invention can be transferred into the host cell by well-known methods, which vary depending on the type of cellular host. For example, calcium chloride transfection is commonly utilized for prokaryotic cells, whereas calcium phosphate treatment or electroporation may be used for other cellular hosts; see Sambrook, supra.

In a still further embodiment, the present invention relates to a cell containing the polynucleotide or vector described above. Preferably, said cell is a eukaryotic, most preferably a mammalian cell if therapeutic uses of the protein are envisaged. Of course, yeast and less preferred prokaryotic, e.g., bacterial cells may serve as well, in particular if the produced protein is used as a diagnostic means.
The polynucleotide or vector of the invention which is present in the host cell may either be integrated into the genome of the host cell or it may be maintained extrachromosomally.
The term "prokaryotic" is meant to include all bacteria which can be transformed or transfected with a DNA or RNA molecules for the expression of a protein of the invention. Prokaryotic hosts may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells. Depending upon the host employed in a recombinant production procedure, the protein encoded by the polynucleotide of the present invention may be glycosylated or may be non-glycosylated. TIRC7 proteins of the invention may also include an initial methionine amino acid residue. A polynucleotide of the invention can be used to transform or transfect the host using any of the techniques commonly known to those of ordinary skill in the art. Furthermore, methods for preparing fused, operably linked genes and expressing them in, e.g., mammalian cells and bacteria are well-known in the art (Sambrook, Molecular Cloning: A Laboratory Manual, Cold Spring Harbor Laboratory, Cold Spring Harbor, NY, 1989). The genetic constructs and methods described therein can be utilized for expression of the TIRC7 protein of the invention in eukaryotic or prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes which are capable of providing phenotypic selection of the transformed cells. Furthermore, transgenic animals, preferably mammals, comprising cells of the invention may be used for the large scale production of the TIRC7 protein of the invention.
Alternatively, an animal, preferably mammalian cell naturally having a polynucleotide of the invention present in its genome can be used and modified such that said cell expresses the endogenus gene corresponding to the polynucleotide of the invention under the control of an heterologous promoter. The introduction of the heterologous promoter which does not naturally control the expression of the polynucleotide of the invention can be done according to standard methods, see supra. Suitable promoter include those mentioned hereinbefore.

Thus, in a further embodiment, the present invention relates to a method for the production of a TIRC7 membrane protein or a biologically active fragment thereof comprising:
(a) culturing a host of the invention under conditions allowing for the expression of the protein; or
(b) in vitro translation of the polynucleotide of the invention;
and recovering the protein produced in (a) or (b).

The transformed hosts can be grown in fermentors and cultured according to techniques known in the art to achieve optimal cell growth. The TIRC7 protein of the invention can then be isolated from the growth medium, cellular lysates, or cellular membrane fractions. Once expressed, the protein of the present invention can be purified according to standard procedures of the art, including ammonium sulfate precipitation, affinity columns, column chromatography, gel electrophoresis and the like; see, Scopes, "Protein Purification", Springer-Verlag, N.Y. (1982). Substantially pure proteins of at least about 90 to 95% homogeneity are preferred, and 98 to 99% or more homogeneity are most preferred, for pharmaceutical uses. Once purified, partially or to homogeneity as desired, the proteins may then be used therapeutically (including extracorporeally) or in developing and performing assay procedures.

Hence, in a still further embodiment, the present invention relates to a TIRC7 membrane protein or a biologically active fragment thereof encoded by the polynucleotide of the invention or produced by a method of as described above. It will be apparent to those skilled in the art that the protein of the invention can be further coupled to other moieties as described above for, e.g., drug targeting and imaging applications. Such coupling may be conducted chemically after expression of the protein to site of attachment or the coupling product may be engineered into the protein of the invention at the DNA level. The DNAs are then expressed in a suitable host system, and the expressed proteins are collected and renatured, if necessary. Furthermore, the provision of the TIRC7 protein of the present invention enables the production of TIRC7 specific antibodies. In this respect, hybridoma technology enables production of cell lines secreting antibody to essentially any desired substance that produces an immune response. RNA encoding the light and heavy chains of the immunoglobulin can then be obtained from the cytoplasm of the hybridoma. The 5' end portion of the mRNA can be used to prepare cDNA to be inserted into an expression vector. The DNA encoding the antibody or its immunoglobulin chains can subsequently be expressed in cells, preferably mammalian cells.
Depending on the host cell, renaturation techniques may be required to attain proper conformation of the antibody. If necessary, point substitutions seeking to optimize binding may be made in the DNA using conventional cassette mutagenesis or other protein engineering methodology such as is disclosed herein.

Thus, the present invention also relates to an antibody specifically recognizing the peptide or polypeptide of the invention.

In a still further embodiment, the present invention relates to a cell that has been modified to express a TIRC7 protein or an antibody of the invention. This embodiment may be well suited for, e.g., restoring T-cell responsiveness to an antigen, in particular if the antibody of the invention capable of stimulating T-cell proliferation is expressed in a form suitable to be presented on the cell surface.

Moreover, the present invention relates to pharmaceutical compositions comprising a peptide or polypeptide being capable of inhibiting T-cell stimulation through the TIRC7 membrane protein and/or being recognized by an antibody capable of inhibiting T-cell stimulation through the TIRC7 membrane protein encoded by a fragment of the above described polynucleotides or an antibody specifically recognizing said peptide or polypeptide. The term "capable of inhibiting T-cell stimulation through TIRC7 membrane protein" denotes the ability of suppressing the proliferation of T-cells in response to alloactivation in a mixed lymphocyte culture or in response to mitogens by way of blocking or antagonizing the biological activity of the TIRC7 membrane protein herein also referred to as TIRC7 protein or TIRC7. The terms "capable of inhibiting T-cell stimulation through TIRC7 membrane protein" and "inhibiting TIRC7 activity" are used interchangeable herein.

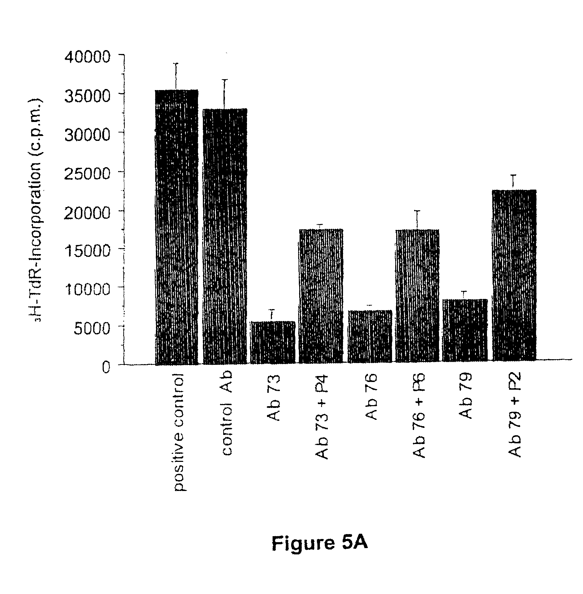

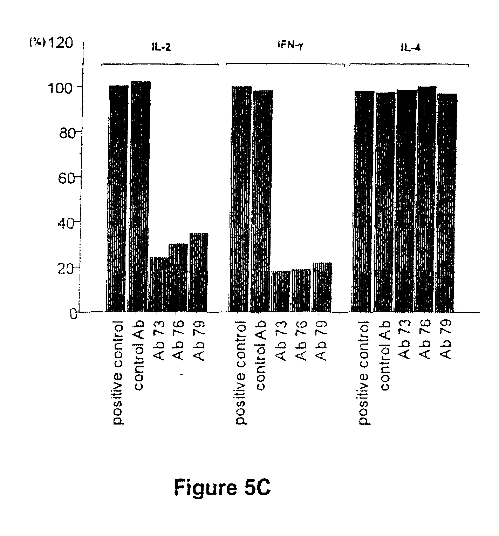

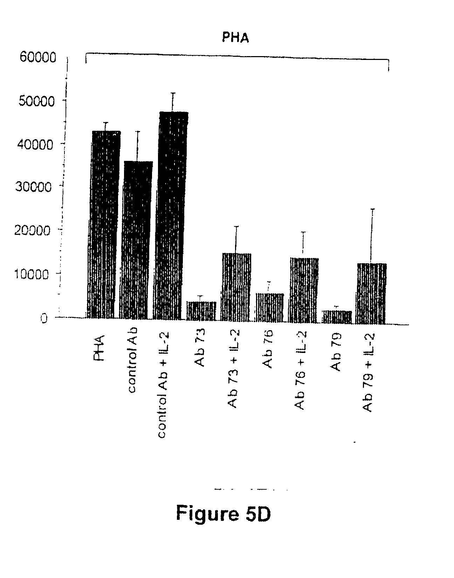

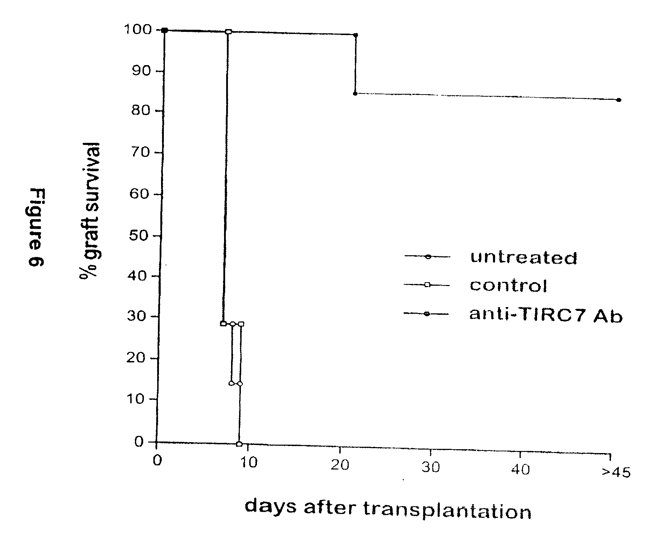

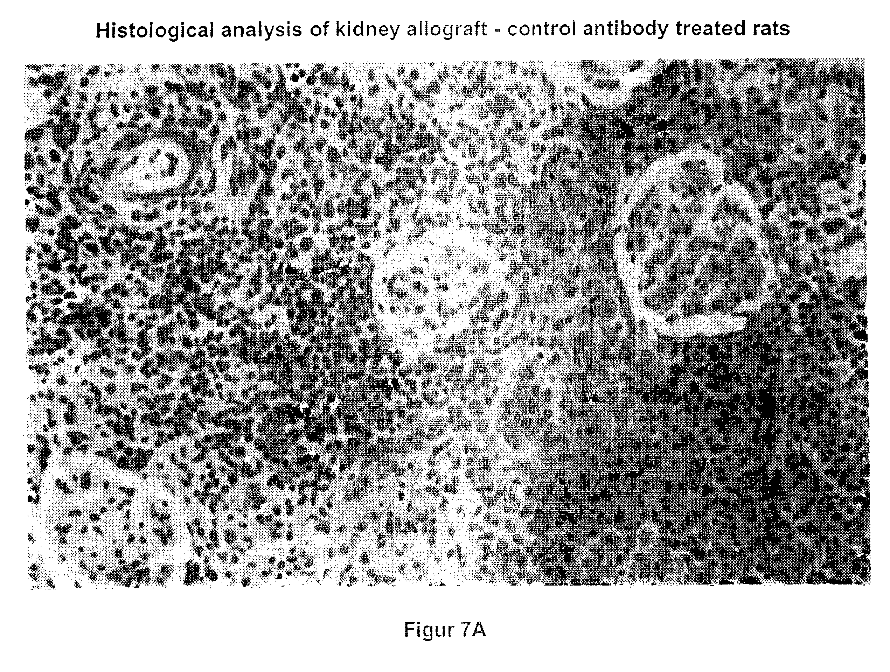

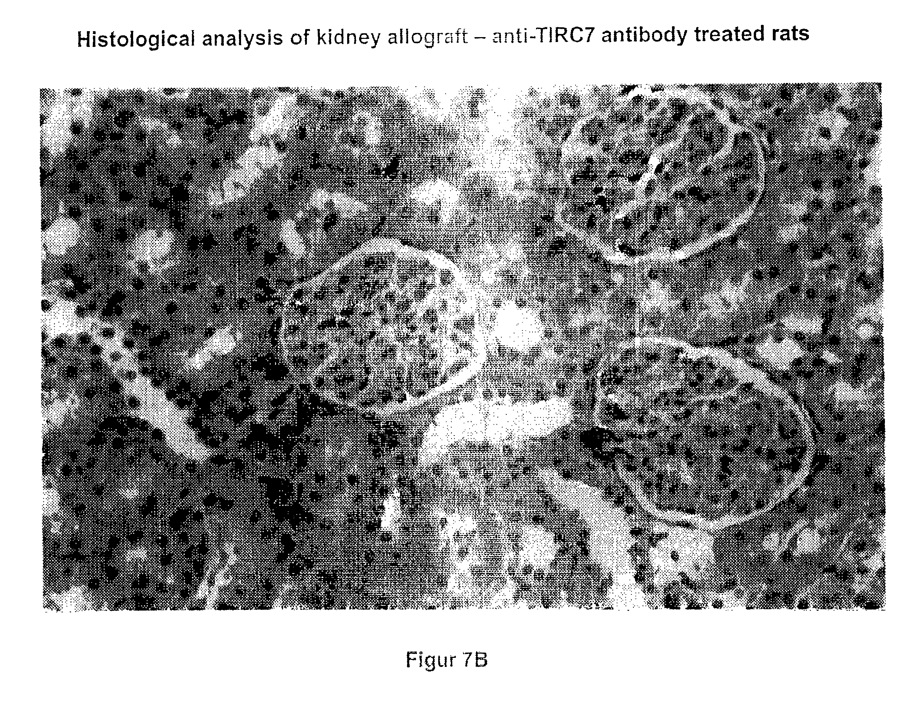

Studies which had been carried out within the scope of the present invention surprisingly revealed that anti-TIRC7 antibodies directed against the extracellular domains, but not those recognizing predicted intracellular domains of the protein, are able to efficiently suppress the proliferation of T-cells in response to alloactivation in a mixed lymphocyte culture or in response to mitogens; see Example 2, Figure 5. Similar results were obtained with in vitro translated soluble TIRC7 protein. The inhibitory effect of anti-TIRC7 antibodies on T-cells induced by a variety of different stimulatory pathways suggests that TIRC7 plays a central role in T cell activation. Moreover, inhibition of T cell proliferation in MLR by antibody targeting of TIRC7 suggest the existence of a ligand specifically interacting with TIRC7. Support for this hypothesis is provided by the dose-dependent inhibition of T cell proliferation in a MLR in the presence of soluble *in vitro* translated TIRC7 protein.
Furthermore, it could be shown in accordance with the present invention that antibody targeting of TIRC7 has a selective inhibitory effect on the Th1 lymphocyte subset, as evidenced by the inhibition of IL-2 and IFN-γ, but not IL-4, cytokine production; see Example 3, Figure 5. With anti-TIRC7 antibody treatment the cells appear to remain in an unresponsive, but functional, state since exogenous recombinant IL-2 reversed the antiproliferative effect of the anti-TIRC7 antibodies. While the above described results hold promise that the novel TIRC7 protein, and antibodies thereto may be therapeutically useful, proof for the concept of the invention, namely the usefulness of the above described compounds for the modulation of the immune response as well as the embodiments derived therefrom and characterized hereinbelow came from further experiments performed in accordance with the present invention demonstrating the ability of an anti-TIRC7 antibody to prevent allograft rejection in the *in vivo* model of rat kidney transplantation; see Example 4, Figures 6 und 7. Moreover, it could be demonstrated in accordance with the present invention that advantageously the effects of antibody targeting of TIRC7 are quite similar to those observed by targeting of costimulatory molecules. Antibody blocking of costimulatory molecules has been shown to inhibit human T cell proliferation (Linsley et al., 1992; Walunas et al., 1994). Furthermore, interruption of CD28/B7 interaction with the soluble protein CTLA4Ig caused inhibition of T cell proliferation (Linsley et al., 1992; Lenschow et al., 1992; Larsen et al., 1996). Further analogy to the effect of TIRC7 antibody targeting is provided by CTLA4Ig selectively blocking Th1 and sparing Th2 lymphocyte responses (Mohammed et al., 1995). It was shown, that administration of CTLA4Ig in an *in vivo* model of kidney allograft transplantation prolonged graft survival (Mohammed et al., 1995) which was similarly observed by TIRC7 antibody targeting in the present Examples. Although these similarities may suggest a costimulatory function, TIRC7 does not share structural or sequence homology with any of the known T cell accessory molecules. Thus, TIRC7 may participitate in a distinct signaling pathway induced early in the course of T cell activation. This possibility is supported by recent reports that interference with pathways mediated by molecules other than the known costimulatory proteins can modulate the T cell response. For example, antibody targeting of the common leukocyte antigen CD45RB was shown to result in a prevention of graft rejection in mice (Lazarovits et al., 1996). Given the functional similarities between TIRC7 and the known T cell accessory molecules, it is expected that the structural novelty of TIRC7 will contribute to the understanding of distinct mechanisms in the T cell response. Moreover, the striking capacity of anti-TIRC7 antibody to significantly prolong allograft survival *in vivo* provide a novel approach for a selective inhibition of undesired T cell activation in human organ transplantation and autoimmune diseases.

In a preferred embodiment of the invention, said peptide or polypeptide encoded by the above described polynucleotide comprises the amino acid sequence depicted in any one of SEQ ID NOS 3 to 9. As is described in the appended examples peptides comprising the above mentioned amino acid sequences correspond to parts of the extracellular domain of the TIRC7 protein and can advantageously be used for the generation of antibodies that are capable of inducing T-cell unresponsiveness.

Accordingly, in a particularly preferred embodiment the pharmaceutical composition of the invention comprises a soluble form of said peptide or polypeptide. In one embodiment, a soluble form of TIRC7 or a TIRC7 ligand is a truncated form of the molecule comprising an extracellular domain of the TIRC7 or a functional portion thereof. A portion of the extracellular domain of TIRC7 which retains the ability to bind to a TIRC7 ligand can be used. Likewise, a portion of the extracellular domain of a TIRC7 ligand which retains the ability to bind to TIRC7 can be used. Another soluble form of TIRC7 or a TIRC7 ligand for use in accordance with the present invention is a fusion protein. The term "fusion protein" as used herein refers to a protein comprised of a first polypeptide from a first protein in contiguous amino acid sequence with a second polypeptide from a second protein. Fusion proteins can be made by standard recombinant DNA techniques wherein a nucleotide sequence encoding the first polypeptide is ligated in-frame to a nucleotide sequence encoding the second polypeptide, and these nucleotide sequences are expressed (e.g., using a recombinant expression vector introduced into a host cell) to produce the fusion protein. A preferred fusion protein is an immunoglobulin fusion protein which includes an extracellular domain, or functional portion of TIRC7 or a TIRC7 ligand linked to an immunoglobulin heavy chain constant region (e.g., the hinge, CH2 and CH3 regions of a human immunoglobulin such as IgG1). Immunoglobulin fusion proteins can be prepared, for example, according to the teachings of Capon, Nature 337 (1989), 525-531.

The antibody comprised in the pharmaceutical composition of the invention preferably has a specificity at least substantially identical to the binding specificity of the natural ligand of the TIRC7 protein of the invention, in particular if T-cell stimulation is desired. Such an antibody can have a binding affinity of at least 10⁵M⁻¹, preferably not higher than 10⁷M⁻¹ if T-cell stimulation is envisaged and advantageously up to 10¹⁰M⁻¹ in case T-cell suppression should be mediated. In a preferred embodiment, antibody
(a) a T-cell suppressive antibody has an affinity of at least about 10⁻⁷ M, preferably at least about 10⁻⁹ M and most preferably at least about 10⁻¹¹ M; and
(b) a T-cell stimulating antibody has an affinity of less than about 10⁻⁷ M, preferably less than about 10⁻⁶ M and most preferably in order of 10⁻⁵M.

In case of bispecific antibodies where one specificity is directed to a target cell to be destroyed, e.g. a tumor cell, it is advantageous if the binding site recognizing the tumor antigen has a high affinity in order to capture the target cells to be destroyed with high efficiency. On the other hand, the binding affinity of the binding site recognizing the TIRC7 protein of the invention should be in the order of those of the natural TIRC7 ligand or of that usually found for the interaction of the T-cell costimulatory molecules with their receptor.

In a preferred embodiment of the invention, said antibody is a monoclonal antibody, a polyclonal antibody, a single chain antibody, a humanized antibody, or fragment thereof that specifically binds said peptide or polypeptide also including a bispecific antibody, a synthetic antibody, an antibody fragment, such as Fab, Fv or scFv fragments etc., or a chemically modified derivative of any of these. Monoclonal antibodies can be prepared, for example, by the techniques as originally described in Köhler and Milstein, Nature 256 (1975), 495, and Galfré, Meth. Enzymol. 73 (1981), 3, which comprise the fusion of mouse myeloma cells to spleen cells derived from immunized mammals with modifications developed by the art. Furthermore, antibodies or fragments thereof to the aforementioned peptides can be obtained by using methods which are described, e.g., in Harlow and Lane "Antibodies, A Laboratory Manual", CSH Press, Cold Spring Harbor, 1988. When derivatives of said antibodies are obtained by the phage display technique, surface plasmon resonance as employed in the BIAcore system can be used to increase the efficiency of phage antibodies which bind to an epitope of the peptide or polypeptide of the invention (Schier, Human Antibodies Hybridomas 7 (1996), 97-105; Malmborg, J. Immunol. Methods 183 (1995), 7-13). The production of chimeric antibodies is described, for example, in WO89/09622. Methods for the production of humanized antibodies are described in, e.g., EP-A1 0 239 400 and WO90/07861. A further source of antibodies to be utilized in accordance with the present invention are so-called xenogenic antibodies. The general principle for the production of xenogenic antibodies such as human antibodies in mice is described in, e.g., WO 91/10741, WO 94/02602, WO 96/34096 and WO 96/33735.

Antibodies to be employed in accordance with the invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook, Molecular Cloning A Laboratory Manual, Cold Spring Harbor Laboratory (1989) N.Y.

The peptides, polypeptides and antibodies comprised in the pharmaceutical compositions of the present invention can comprise a further domain, said domain being linked by covalent or non-covalent bonds. The linkage can be based on genetic fusion according to the methods known in the art and described above or can be performed by, e.g., chemical cross-linking as described in, e.g., WO 94/04686. The additional domain present in the fusion protein comprising the peptide, polypeptide or antibody employed in accordance with the invention may preferably be linked by a flexible linker, advantageously a polypeptide linker, wherein said polypeptide linker comprises plural, hydrophilic, peptide-bonded amino acids of a length sufficient to span the distance between the C-terminal end of said further domain and the N-terminal end of the peptide, polypeptide or antibody or vice versa. The above described fusion protein may further comprise a cleavable linker or cleavage site for proteinases.

Furthermore, said further domain may be of a predefined specificity or function. For example, the literature contains a host of references to the concept of targeting bioactive substances such as drugs, toxins, and enzymes to specific points in the body to destroy or locate malignant cells or to induce a localized drug or enzymatic effect. It has been proposed to achieve this effect by conjugating the bioactive substance to monoclonal antibodies (see, e.g., N.Y. Oxford University Press; and Ghose, (1978) J. Natl. Cancer Inst. 61:657-676).
In this context, it is understood that the polypeptides present in the pharmaceutical composition according to the invention may be further modified by conventional methods known in the art. This allows for the construction of fusion proteins comprising the peptide, polypeptide or antibody of the invention and other functional amino acid sequences, e.g., nuclear localization signals, transactivating domains, DNA-binding domains, hormone-binding domains, protein tags (GST, GFP, h-myc peptide, FLAG, HA peptide) which may be derived from heterologous proteins. Thus, administration of the composition of the invention can utilize unlabeled as well as labeled (poly)peptides or antibodies.
For example, the peptides, polypeptides and antibodies can be administered labeled with a therapeutic agent. These agents can be coupled either directly or indirectly to the antibodies or (poly)peptides of the invention, see supra, and can be selected to enable drug release from the antigen at the target site. Examples of therapeutic agents which can be coupled to the (poly)peptides and antibodies for immunotherapy are drugs, radioisotopes, lectins, and toxins. The drugs which can be conjugated to the polypeptides of the invention include compounds which are classically referred to as drugs such as mitomycin C, daunorubicin, and vinblastine. In using radioisotopically conjugated (poly)peptides or antibodies of the invention for, e.g., immunotherapy, certain isotopes may be more preferable than others depending on such factors as leukocyte distribution as well as stability and emission. Depending on the autoimmune response, some emitters may be preferable to others. In general, α and β particle-emitting radioisotopes are preferred in immunotherapy. Preferred are short range, high energy α emitters such as ²¹²Bi. Examples of radioisotopes which can be bound to the (poly)peptides and antibodies of the invention for therapeutic purposes are ¹²⁵I, ¹³¹I, ⁹⁰Y, ⁶⁷Cu, ²¹²Bi, ²¹²At, ²¹¹Pb, ⁴⁷Sc, ¹⁰⁹Pd and ¹⁸⁸Re.
Lectins are proteins, usually isolated from plant material, which bind to specific sugar moieties. Many lectins are also able to agglutinate cells and stimulate lymphocytes. However, ricin is a toxic lectin which has been used immunotherapeutically. This is accomplished by binding the α-peptide chain of ricin, which is responsible for toxicity, to the polypeptide to enable site specific delivery of the toxic effect.
Toxins are poisonous substances produced by plants, animals, or microorganisms that, in sufficient dose, are often lethal. Diphtheria toxin is a substance produced by Corynebacterium diphtheria which can be used therapeutically. This toxin consists of an α and β subunit which under proper conditions can be separated. The toxic A component can be bound to an antibody of the invention and be used for site specific delivery to the interacting T-cell.
Other therapeutic agents such as described above which can be coupled to the polypeptide of the invention, as well as corresponding ex vivo and in vivo therapeutic protocols, are known, or can be easily ascertained, by those of ordinary skill in the art.
Wherever appropriate the person skilled in the art may use a polynucleotide of the invention described hereinbelow encoding any one of the above described (poly)peptides and antibodies, respectively, or the corresponding vectors instead of the proteinaeous material itself.

Thus, the person skilled in the art will readily appreciate that the (poly)peptide and antibody of the invention can be used for the construction of fusion proteins of desired specificity and biological function. The (poly)peptides and antibodies then optionally employed in accordance with the present invention of the invention as well as fusion protein thereof are expected to play an important therapeutic and scientific role in particular in the medical field, for example, in the development of new treatment approaches for T-cell related disorders such as certain forms of cancer and autoimmune diseases or as interesting tools for the analysis and modulation of the corresponding cellular signal transduction pathways.

Preferably said further domain comprises a molecule selected from the group consisting of effector molecules having a conformation suitable for biological activity, amino acid sequences capable of sequestering an ion, and amino acid sequences capable of selective binding to a solid support or to a preselected antigen. Said domain may comprises an enzyme, toxin, receptor, binding site, biosynthetic antibody binding site, growth factor, cell-differentiation factor, lymphokine, cytokine, hormone, a remotely detectable moiety, anti-metabolite, a radioactive atom or an antigen. Said antigen can be, e.g., tumor antigen, a viral antigen, a microbial antigen, an allergen, an auto-antigen, a virus, a microorganism, a polypeptide, a peptide or a plurality of tumor cells. Furthermore, said sequence capable of sequestering an ion includes calmodulin, methallothionein, a fragment thereof, or an amino acid sequence rich in at least one of glutamic acid, aspartic acid, lysine, and arginine. In addition, said polypeptide sequence capable of selective binding to a solid support can be a positively or negatively charged amino acid sequence, a cysteine-containing amino acid sequence, avidin, streptavidin, a fragment of Staphylococcus protein A, GST, a His-tag, a FLAG-tag, Lex A or c-myc as used in the appended examples. Some of the effector molecules and amino acid sequences described above may be present in a preform which itself is either active or not and which may be removed, when, e.g., entering a certain cellular environment.
The pharmaceutical composition of the present invention may further comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g.. by intravenous, intraperitoneal, subcutaneous, intramuscular, topical or intradermal administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 µg (or of nucleic acid for expression or for inhibition of expression in this range); however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. Dosages will vary but a preferred dosage for intravenous administration of DNA is from approximately 10⁶ to 10¹² copies of the DNA molecule. The compositions of the invention may be administered locally or systemically. Administration will generally be parenterally, e.g., intravenously; DNA may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents such as T-cell costimulatory molecules or cytokines known in the art, or their inhibitors or activators depending on the intended use of the pharmaceutical composition.

Furthermore, it is envisaged by the present invention that the various polynucleotides and vectors encoding the above described peptides or polypeptides are administered either alone or in any combination using standard vectors and/or gene delivery systems, and optionally together with a pharmaceutically acceptable carrier or excipient. For example, the polynucleotide of the invention can be used alone or as part of a vector to express the (poly)peptide of the invention in cells, for, e.g., gene therapy or diagnostics of diseases related to T-cell disorders. The polynucleotides or vectors of the invention are introduced into the cells which in turn produce the (poly)peptide. Subsequent to administration, said polynucleotides or vectors may be stably integrated into the genome of the subject. On the other hand, viral vectors may be used which are specific for certain cells or tissues and persist in said cells. Suitable pharmaceutical carriers and excipients are well known in the art. The pharmaceutical compositions prepared according to the invention can be used for the prevention or treatment or delaying of different kinds of diseases, which are related to T-cell related immunodeficiencies and malignancies.

Furthermore, it is possible to use a pharmaceutical composition of the invention which comprises a polynucleotide or vector of the invention in gene therapy. Gene therapy, which is based on introducing therapeutic genes into cells by ex-vivo or in-vivo techniques is one of the most important applications of gene transfer. Suitable vectors and methods for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO94/29469; WO 97/00957 or Schaper, Current Opinion in Biotechnology 7 (1996), 635-640, and references cited therein. The polynucleotides and vectors of the invention may be designed for direct introduction or for introduction via liposomes, or viral vectors (e.g. adenoviral, retroviral) into the cell. Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell populations. Preferably, said cell is a germ line cell, embryonic cell, or egg cell or derived therefrom, most preferably said cell is a stem cell. As mentioned above, suitable gene delivery systems may include liposomes, receptor-mediated delivery systems, naked DNA, and viral vectors such as herpes viruses, retroviruses, adenoviruses, and adeno-associated viruses, among others. Delivery of nucleic acids to a specific site in the body for gene therapy may also be accomplished using a biolistic delivery system, such as that described by Williams (Proc. Natl. Acad. Sci. USA 88 (1991), 2726-2729). It is to be understood that the introduced polynucleotides and vectors express the gene product after introduction into said cell and preferably remain in this status during the lifetime of said cell. For example, cell lines which stably express the polynucleotide under the control of appropriate regulatory sequences may be engineered according to methods well known to those skilled in the art. Rather than using expression vectors which contain viral origins of replication, host cells can be transformed with the polynucleotide of the invention and a selectable marker, either on the same or separate plasmids. Following the introduction of foreign DNA, engineered cells may be allowed to grow for 1-2 days in an enriched media, and then are switched to a selective media. The selectable marker in the recombinant plasmid confers resistance to the selection and allows for the selection of cells having stably integrated the plasmid into their chromosomes and grow to form foci which in turn can be cloned and expanded into cell lines. Such engineered cell lines are also particularly useful in screening methods for the detection of compounds involved in, e.g., T-cell activation or stimulation.
A number of selection systems may be used, including but not limited to the herpes simplex virus thymidine kinase (Wigler, Cell 11 (1977), 223), hypoxanthine-guanine phosphoribosyltransferase (Szybalska, Proc. Natl. Acad. Sci. USA 48 (1962), 2026), and adenine phosphoribosyltransferase (Lowy, Cell 22 (1980), 817) in tk⁻, hgprt⁻ or aprt⁻ cells, respectively. Also, antimetabolite resistance can be used as the basis of selection for dhfr, which confers resistance to methotrexate (Wigler, Proc. Natl. Acad. Sci. USA 77 (1980), 3567; O'Hare, Proc. Natl. Acad. Sci. USA 78 (1981), 1527), gpt, which confers resistance to mycophenolic acid (Mulligan, Proc. Natl. Acad. Sci. USA 78 (1981), 2072); neo, which confers resistance to the aminoglycoside G-418 (Colberre-Garapin, J. Mol. Biol. 150 (1981), 1); hygro, which confers resistance to hygromycin (Santerre, Gene 30 (1984), 147); or puromycin (pat, puromycin N-acetyl transferase). Additional selectable genes have been described, for example, trpB, which allows cells to utilize indole in place of tryptophan; hisD, which allows cells to utilize histinol in place of histidine (Hartman, Proc. Natl. Acad. Sci. USA 85 (1988), 8047); and ODC (omithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.).

In another embodiment the present invention relates to a diagnostic composition comprising any one of the above described proteins, antibodies, (poly)peptides, polynucleotides, vectors or cells and optionally suitable means for detection. The (poly)peptides and antibodies described above are, for example, suited for use in immunoassays in which they can be utilized in liquid phase or bound to a solid phase carrier. Examples of immunoassays which can utilize said (poly)peptide are competitive and non-competitive immunoassays in either a direct or indirect format. Examples of such immunoassays are the radioimmunoassay (RIA), the sandwich (immunometric assay) and the Western blot assay. The (poly)peptides and antibodies can be bound to many different carriers and used to isolate cells specifically bound to said polypeptides. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention.
There are many different labels and methods of labeling known to those of ordinary skill in the art. Examples of the types of labels which can be used in the present invention include enzymes, radioisotopes, colloidal metals, fluorescent compounds, chemiluminescent compounds, and bioluminescent compounds; see also the embodiments discussed hereinabove.

Said diagnostic compositions may also be used for methods for detecting expression of a polynucleotide of the invention by detecting the presence of mRNA coding for a TIRC7 membrane protein which comprises obtaining mRNA from a cell and contacting the mRNA so obtained with a probe comprising a nucleic acid molecule of at least 15 nucleotides capable of specifically hybridizing with a polynucleotide of the invention under suitable hybridizing conditions (see also supra), detecting the presence of mRNA hybridized to the probe, and thereby detecting the expression of the TIRC7 protein by the cell.
Furthermore, the invention comprises methods of detecting the presence of a TIRC7 membrane protein in a sample, for example, a cell sample, which comprises obtaining a cell sample from a subject, contacting said sample with one of the aforementioned antibodies under conditions permitting binding of the antibody to the TIRC7 protein, and detecting the presence of the antibody so bound, for example, using immuno assay techniques such as radioimmunoassay or enzymeimmunoassay. Furthermore, one skilled in the art may specifically detect and distinguish polypeptides which are functional TIRC7 proteins from mutated forms which have lost or altered their T-cell stimulatory activity by using an antibody which either specifically recognizes a (poly)peptide which has TIRC7 activity but does not recognize an inactive form thereof or which specifically recognizes an inactive form but not the corresponding polypeptide having TIRC7 activity. The antibodies of the present invention may also be used in affinity chromatography for purifying the TIRC7 membrane protein or above described (poly)peptides and isolating them from various sources.
The invention also encompasses a method for diagnosing in a subject a predisposition to a disorder associated with the expression of a TIRC7 allele which comprises isolating DNA from victims of the the disorder associated with the under-or over-expression of a TIRC7 protein; digesting the isolated DNA with at least one restriction enzyme: electrophoretically separating the resulting DNA fragments on a sizing gel; contacting the resulting gel with a nucleic acid probe as described above capable of specifically hybridizing to DNA encoding a TIRC7 protein and iabeled with a detectable marker; detecting labeled bands on the gel which have hybridized to the labeled probe to create a band pattern specific to the DNA of victims of the disorder associated with the expression of a TIRC7 protein; preparing the subject's DNA according to the above-mentioned steps to produce detectable labeled bands on a gel; and comparing the band pattern specific to the DNA of victims of the disorder associated with the expression of a TIRC7 protein and the subject's DNA to determine whether the patterns are the same or different and to diagnose thereby predisposition to the disorder if the patterns are the same. The detectable markers of the present invention may be labeled with commonly employed radioactive labels, such as, for example, ³²P and ³⁵S, although other labels such as biotin or mercury as well as those described above may be employed as well. Various methods well-known to the person skilled in the art may be used to label the detectable markers. For example, DNA sequences and RNA sequences may be labeled with ³²P or ³⁵S using the random primer method. Once a suitable detectable marker has been obtained, various methods well-known to the person skilled in the art may be employed for contacting the detectable marker with the sample of interest. For example, DNA-DNA, RNA-RNA and DNA-RNA hybridizations may be performed using standard procedures. Various methods for the detection of nucleic acids are well-known in the art, e.g., Southern and northern blotting, PCR, primer extension and the like. Furthermore, the mRNA, cRNA, cDNA or genomic DNA obtained from the subject may be sequenced to identify mutations which may be characteristic fingerprints of TIRC7 mutations in disorders associated with the expression of TIRC7 or mutated versions thereof. The present invention further comprises methods, wherein such a fingerprint may be generated by RFLPs of DNA or RNA obtained from the subject, optionally the DNA or RNA may be amplified prior to analysis, the methods of which are well known in the art. RNA fingerprints may be performed by, for example, digesting an RNA sample obtained from the subject with a suitable RNA-Enzyme, for example RNase T₁, RNase T₂ or the like or a ribozyme and, for example, electrophoretically separating and detecting the RNA fragments on PAGE as described above or in the appended examples.

In a preferred embodiment, the pharmaceutical composition of the present invention comprises at least one second agent, preferably an agent which inhibits or activates T-cell stimulation depending on the intended use. Such agents include, for example, molecules that are capable of blocking or mimicking receptor/ligand interaction or the like which leads to T-cell suppression and activation, respectively.
Such agents comprise those blocking the activity of, e.g., costimulatory molecules, integrins, lg-superfamily molecules, selectins as well as drugs blocking chemokines and their respective receptor interactions, drugs blocking IL2/IL2-receptor interaction and other conventional immunosuppressive drugs such as IL-2R mAbs, IL-Toxins and IL-Muteins. Examples for costimulatory molecules and their ligands are described in the prior art, e.g., in Schwartz, Cell 71 (1992), 1065-1068. The interruption of the receptor/ligand interactions by using mAbs or soluble CTLA4Ig for the interaction between CD28 to the B7-2 and CTLA4 to B7-1 and B7-2 are described in Blazar, J. Immunol. 157 (1996), 3250-3259; Bluestone, Immunity 2 (1995), 555-559; Linsley, Science 257 (1992), 792-95. Examples for blocking the receptor/ligand interaction by using mAbs to CD40 or CD40L are reported by Burden, Nature 381 (1996), 434-435; Kirk, Proc. Natl. Acad. Sci. USA 94 (1997), 8789-8794. CD2 antigen and its ligand LFA-3 are described in Bagogui Li et al., review in Adhesion Molecules, Fusion proteins, Novel Peptides, and Monoclonal Antibodies, Recent Developments in Transplantation Medicine, Vol. II, 1995, Physicians&Scientists Publishing Co., Inc. and blocking of their interaction by using of mAbs (anti-Leu-5b, OKT11, T11) is reported in Brumberg, Transplantation 51 (1991) 219-225 or CD2.IgG1 fusion protein. The use of monoclonal Abs agains CD4 molecule is described in Cosimi, Surgery 108 (1990), 406-414. CD47 blockade by mAbs is described by Rheinhold, J. Exp. Med. 185 (1997), 1-11. Integrins and ig-superfamily molecules include LFA-1 with its ligand ICAM-1, -2, -3, Mac-1 with ist ligand ICAM-1, -3; ICAM-1 with its ligand LFA-1, Mac-1, CD43; ICAM-2 with ist ligand LFA-1; ICAM-3 with its ligand LFA-1, Mac-1; VLA4 and VCAM-1 see, e.g., David, Adams, review in Adhesion Molecules, Fusion proteins, Novel Peptides, and Monoclonal Antibodies, Recent Developments in Transplantation Medicine, Vol. II, 1995. Physicians&Scientists Publishing Co., Inc.; Isobe, Science, 255 (1992), 1125-1127; Cosimi, J. Immunology 144 (1990), 4604-4612; Hynes, Cell 69 (1992), 11-25. Furthermore selectively interfering agents with VLA-4 mAbs to the alpha4 integrin chain (CD49d) can be used, beta1 integrin chain (CD29), or an activation - induced neo-epitope of VLA-4 as well as soluble VLA-4 ligands such as soluble fibronectin or its relevant peptide (GPEILDVPST), or soluble VCAM-1 or its relevant peptide. More selectively blocking agents are antisense oligonucleotides, designed to selectively hybridize with cytoplasmic alpha4, beta1, or VCAM-1 mRNA; Fedoseyeva, J. Immunol. 57 (1994), 606-612.
Another example is the drug pentoxifylline (PTX) that is able to block expression of VCAM-1; Besler, J. Leukoc. Biol. 40 (1986), 747-754. Furthermore, VCAM-1 mAb, M/K-2, anti-murine, for example could prolong allograft survival, Orosz, Transplantation, 56 (1993), 453-460.
Blocking of members of the integrin family and their ligands by using mAbs is decribed in Kupiec-Weglinski, review in Adhesion Molecules, Fusion proteins, Novel Peptides, and Monoclonal Antibodies, Recent Developments in Transplantation Medicine, Vol. II, 1995, Physicians&Scientists Publishing Co., Inc. Selectins, e.g., L-selectin (CD62L), E-selectin (CD62E), P-selectin (CD62P) have been described in Forrest and Paulson, Selectin family of adhesion molecules. In: Granger and Schmid-Schonbein, eds. Physiology and Pathophysiology of Leukocyte Adhesion. New York, Oxford Press, 1995, pp 68-146.
The combination of conventional immunosuppressive drugs, e.g., ATG, ALG, OKT3, Azathioprine, Mycophenylate, Mofetyl, Cyclosporin A, FK506, Corticosteroids may be used as decribed in Cosimi, Transplantation 32 (1981), 535-539; Shield, Transplantation 38 (1984), 695-701.
The interruption of chemokines and interactions with their respective receptor by using mAbs is reviewed in Luster, Chemokines-chemotactic cytokines that mediate inflammation, New Engl. J. Med. Feb. (1998), 436-445.

Thus, any agent as defined above and referenced by way of example can be used in accordance with the pharmaceutical composition of the invention or the methods and uses described hereinbelow. On the other hand, it is evident to the person skilled in the art that the polynucleotides, vectors, proteins, peptides, polypeptides, antibodies, cells, and pharmaceutical compositions of the invention can be used for methods and uses described for the above referenced T-cell costimulatory molecules, inhibitors and drugs.

Advantageously, the pharmaceutical composition of the invention is intended for use in organ transplantation, for the treatment of autoimmune, allergic or infectious diseases, or for the treatment of tumors. An example for the use of the pharmaceutical composition of the invention for improving allograft or xenograft tolerance is described with respect to administration of an LFA-3 and CD2 binding protein, respectively, in WO93/06852.

Furthermore, this invention pertains to methods for modulating (antigen-specific) T-cell unresponsiveness. The term "T-cell unresponsiveness" as used herein refers to a reduction in or lack of T-cell proliferation, lymphokine secretion or induction of effector functions by a T-cell upon exposure to the antigen (or antigenic portion). The pharmaceutical compositions of this invention provide a means for inducing, maintaining or reversing unresponsiveness of a T-cell to an antigen *in vitro* or *in vivo*. Accordingly, the compounds of this invention are particularly useful for modulating (antigen-specific) T-cell unresponsiveness. The term "modulation" is intended to include both inducing and maintenance of an unresponsive state and reversal of an unresponsive state, i.e., restoration of T-cell responsiveness.

As is described in Example 3, after anti-TIRC7 antibody treatment T-cells remain in an unresponsive, but functional state since exogenous recombinant IL-2 reversed the antiproliferative effect of the antibodies. Thus, in another embodiment the present invention relates to an in vitro method for inducing or maintaining unresponsiveness of a T-cell to an antigen comprising contacting the T-cell with an agent which inhibits stimulation of the T-cell through a TIRC7 membrane protein. Recipes for how to carry out methods for modulation T-cell unresponsiveness are generally known to the skilled person and are described in, e.g., WO95/24217 and references cited therein.

Hence, the present invention also relates to the use of an agent which inhibits T-cell stimulation through a TIRC7 membrane protein for the preparation of a pharmaceutical composition for inducing or maintaining T-cell unresponsiveness to an antigen in a subject. The methods and uses of the invention may be used with primed or unprimed T-cells depending on what is intended by the person skilled in the art.

Preferably, in the method or the use of the invention the agent blocks an interaction of the TIRC7 membrane protein with its ligand. As is described above, the results of the experiments performed within the scope of the present invention suggest the existence of a ligand interacting with the TIRC7 protein and thereby stimulating T-cell proliferation. Blocking said interaction, e.g. with antibodies or soluble (poly)peptides derived from the TIRC7 membrane protein should result in T cell unresponsiveness. Thus, in a preferred embodiment of the method or the use of the invention the agent is a polynucleotide, a vector, a cell, peptide or polypeptide, or antibody described hereinbefore.

In another preferred embodiment of the invention, the above described method or use further comprise the use of a second agent as defined above.

In another embodiment, the present invention relates to a pharmaceutical composition comprising a first agent which stimulates a T-cell through a TIRC7 membrane protein, and optionally a pharmaceutically acceptable carrier. As is immediately evident to the person skilled in the art, the provision of the novel TIRC7 protein of the invention opens up the way of alternative approaches for T-cell stimulation and treating corresponding diseases. The agent that stimulates the T-cell through the TIRC7 membrane protein is expected to markedly enhance the proliferation of (activated) T-cells and thus is capable of augmenting the immune response. Examples for this type of "Vaccine" is described, e.g., in WO91/11194 and in the literature, e.g., referred to above. Such agents also comprise promoters which can be inserted in front of the coding region of the TIRC7 protein encoding gene, e.g., via gene transfer and homologous recombination in the 5' untranslated region of the gene, see also supra. Such promoter may be regulated and thus permit the controlled expression of the TIRC7 protein in certain cells.

Preferably, said agent is a ligand of the TIRC7 membrane protein or is at least one anti-TIRC7 membrane protein antibody described above.

In a preferred embodiment of the invention, the pharmaceutical composition further comprises a second agent which stimulates T-cell proliferation, for example IL-2, IL-4 or an agent which stimulates a T-cell through a CD2, CD28, CD40 or CTLA4 surface receptor.

In a further embodiment the present invention relates to an in vitro method for restoring responsiveness to an antigen by a T-cell which is unresponsive to the antigen, comprising contacting the T-cell in the presence of the antigen with a first agent which stimulates the T-cell through a TIRC7 membrane protein. Besides the application of the ligand itself said TIRC7 ligand may be preferably expressed on the cell surface by introducing into the cell a nucleic acid molecule encoding the TIRC7 ligand in a suitable form for expression of the TIRC7 ligand on the cell surface. Preferably the cell is a tumor cell. Vectors and methods for the introduction of such, nucleic acid molecules are well known to the skilled person and are also described, e.g., above.

The method of the invention can further comprise contacting the T-cell with a second agent as defined for the pharmaceutical compositions above. Preferably, the T-cell is contacted with the second agent prior to being contacted with the first agent.

in a preferred embodiment of the method of the invention, the second agent, preferably CD2, CD28, CTLA4 ligand or CD40 is expressed on the cell surface by introducing into the cell a nucleic acid molecule encoding the CD2, CD28, CTLA4 ligand or CD40 in a form suitable for expression of said ligand on the cell surface.

In another embodiment, the present invention relates to the use of a first agent as defined above, which is capable of stimulating a T-cell through a TIRC7 membrane protein for the preparation of a pharmaceutical composition for stimulating a T-cell response to a tumor cell in a subject with a tumor. Such agents comprise, for example, also bispecific antibodies described supra. Preferably the tumor cell is modified to express a TIRC7 ligand and/or a CD2, CD28, CTLA4 ligand or CD40 preferably on the tumor cell surface.

In a preferred embodiment of the use the invention the tumor cell is obtained from the subject, modified ex vivo to form a modified tumor cell and said modified tumor cell is used for the preparation of a pharmaceutical composition which is designed for administration to the subject.

In another preferred embodiment of the use of the invention the T-cells are obtained from a subject, contacted with IL-2 or IL-4 ex vivo and said modified T-cells are used for the preparation of pharmaceutical composition which is designed for the administration to the subject.

In a particularly preferred embodiment of the invention, the uses, methods and pharmaceutical compositions are intended to be applied to a subject who is a recipient of a bone marrow or peripheral stem cell transplant. Preferably, the pharmaceutical composition is designed to be contacted with bone marrow or peripheral stem cells prior to transplantation into the recipient.

In a further particular preferred embodiment, the methods and uses of the present invention are applied in organ graft transplantation, peripheral stem cell transplantation or for the treatment of auto-immune diseases or allergy.

Furthermore, the invention relates to a method for identifying T-cell activating or co-stimulating compounds or for identifying inhibitors of T-cell activation and stimulation comprising
(a) culturing T-cells in the presence of the TIRC7 protein, (poly)peptide, antibody, or cell described above and, optionally, in the presence of a component capable of providing a detectable signal in response to T-cell activation, with a compound to be screened under conditions permitting interaction of the compound with the TIRC7 protein, (poly)peptide, antibody or cell(s); and
(b) detecting the presence or absence of a signal generated from the interaction of the compound with the cells.

The term "compound" in the method of the invention includes a single substance or a plurality of substances which may or may not be identical.
Said compound(s) may be comprised in, for example, samples, e.g., cell extracts from, e.g., plants, animals or microorganisms. Furthermore, said compounds may be known in the art but hitherto not known to be capable of inhibiting T-cell activation or not known to be useful as a T-cell costimulatory factor, respectively. The plurality of compounds may be, e.g., added to the culture medium or injected into the cell.
If a sample containing (a) compound(s) is identified in the method of the invention, then it is either possible to isolate the compound from the original sample identified as containing the compound in question or one can further subdivide the original sample, for example, if it consists of a plurality of different compounds, so as to reduce the number of different substances per sample and repeat the method with the subdivisions of the original sample. It can then be determined whether said sample or compound displays the desired properties by methods known in the art such as described herein and in the appended examples. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. The methods of the present invention can be easily performed and designed by the person skilled in the art, for example in accordance with other cell based assays described in the prior art (see, e.g., EP-A-0 403 506) or by using and modifying the methods as described in the appended examples. Furthermore, the person skilled in the art will readily recognize which further compounds and/or cells may be used in order to perform the methods of the invention, for example, B-cells, interleukins, or enzymes, if necessary, that, e.g., convert a certain precursor into the compound which in turn stimulates or suppresses T-cell activation or that provide for (co)stimulatory signals. Such adaptation of the method of the invention is well within the skill of the person skilled in the art and can be performed without undue experimentation.

Compounds which can be used in accordance with the method of the present invention include peptides, proteins, nucleic acids including cDNA expression libraries, antibodies, small organic compounds, ligands, peptidomimetics, PNAs and the like. Said compounds can also be functional derivatives or analogues of known T-cell activators or inhibitors. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art or as described, for example, in the appended examples. Furthermore, peptidomimetics and/or computer aided design of appropriate activators or inhibitors of T-cell activation can be used, for example, according to the methods described below. Appropriate computer programs can be used for the identification of interactive sites of a putative inhibitor and the TIRC7 protein by computer assistant searches for complementary structural motifs (Fassina, Immunomethods 5 (1994), 114-120). Further appropriate computer systems for the computer aided design of protein and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used in combination with the method of the invention for, e.g., optimizing known T-cell activators or inhibitors. Appropriate peptidomimetics can also be identified by the synthesis of peptidomimetic combinatorial libraries through successive chemical modification and testing the resulting compounds, e.g., according to the methods described herein and in the appended examples. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of inhibitors or activators of T-cell stimulation can be used for the design of peptidomimetic inhibitors or activators of T-cell activation to be tested in the method of the invention (Rose, Biochemistry 35 (1996), 12933-12944; Rutenber, Bioorg. Med. Chem. 4 (1996), 1545-1558).

In summary, the present invention provides methods for identifying compounds which are capable of modulating T-cell mediated immune responses. Accordingly compounds identified in accordance with the method of the present invention to be inhibitors and activators, respectively, of T-cell stimulation or activation are also within the scope of the present invention.
Compounds found to activate T-cell mediated responses may be used in the treatment of cancer and related diseases. In addition, it may also be possible to specifically inhibit viral diseases, thereby preventing viral infection or viral spread. Compounds identified as suppressors of T-cell activation or stimulation can be used, e.g., for treating skin conditions (see, e.g., WO93/06866) or in allogenic or xenogenic cell or organ transplantation in order to avoid graft rejection; see also supra.
The compounds identified or obtained according to the method of the present invention are thus expected to be very useful in diagnostic and in particular for therapeutic applications.
Hence, in a further embodiment the invention relates to a method for the production of a pharmaceutical composition comprising formulating and optionally synthesizing the compound identified in step (b) of the above described method of the invention in a pharmaceutically acceptable form. Hence, the present invention generally relates to a method of making a therapeutic agent comprising synthesizing the proteins, (poly)peptides, polynucleotides, vectors, antibodies or compounds according to the invention in an amount sufficient to provide said agent in a therapeutically effective amount to the patient. Methods for synthesizing these agents are well known in the art and are described, e.g. above.
The therapeutically useful compounds identified according to the method of the invention may be administered to a patient by any appropriate method for the particular compound, e.g., orally, intravenously, parenterally, transdermally, transmucosally, or by surgery or implantation (e.g., with the compound being in the form of a solid or semi-solid biologically compatible and resorbable matrix) at or near the site where the effect of the compound is desired. Therapeutic doses are determined to be appropriate by one skilled in the art, see also supra.

Such useful compounds can be for example transacting factors which bind to the TIRC7 protein of the invention. Identification of transacting factors can be carried out using standard methods in the art (see, e.g., Sambrook, supra, and Ausubel, supra). To determine whether a protein binds to the TIRC7 protein of the invention, standard native gel-shift analyses can be carried out. In order to identify a transacting factor which binds to the TIRC7 of the invention, the polypeptides and peptides of the invention can be used as an affinity reagent in standard protein purification methods, or as a probe for screening an expression library. Once the transacting factor is identified, modulation of its binding to the TIRC7 protein of the invention can be pursued, beginning with, for example, screening for inhibitors against the binding of the transacting factor to the TIRC7 protein of the present invention. Activation or repression of TIRC7 specific genes could then be achieved in subjects by applying the transacting factor (or its inhibitor) or the gene encoding it, e.g., in a vector described in the embodiments hereinbefore. In addition, if the active form of the transacting factor is a dimer, dominant-negative mutants of the transacting factor could be made in order to inhibit its activity. Furthermore, upon identification of the transacting factor, further components in the pathway leading to activation (e.g. signal transduction) or repression of a gene encoding the TIRC7 protein of the present invention can then be identified. Modulation of the activities of these components can then be pursued, in order to develop additional drugs and methods for modulating the expression or activity of the TIRC7 protein of the present invention.

Beside the above described possibilities to use the polynucleotides according to the invention for gene therapy and their use to identify homologous molecules, the described polynucleotides may also be used for several other applications, for example, for the identification of nucleic acid molecules which encode proteins which interact with the TIRC7 protein described above. This can be achieved by assays well known in the art, for example, as described in Scofield (Science 274 (1996), 2063-2065) by use of the so-called yeast "two-hybrid system". In this system the (poly)peptide encoded by the polynucleotides according to the invention or a smaller part thereof is linked to the DNA-binding domain of the GAL4 transcription factor. A yeast strain expressing this fusion protein and comprising a lacZ reporter gene driven by an appropriate promoter, which is recognized by the GAL4 transcription factor, is transformed with a library of cDNAs which will express animal, preferably mammal proteins or peptides thereof fused to an activation domain. Thus, if a peptide encoded by one of the cDNAs is able to interact with the fusion protein comprising a (poly)peptide of the invention, the complex is able to direct expression of the reporter gene. In this way the polynucleotide according to the invention and the encoded peptide can be used to identify peptides and proteins interacting with TIRC7 proteins. Other methods fcr identifying compounds which interact with the TIRC7 protein according to the invention or nucleic acid molecules encoding such molecules are, for example, the in vitro screening with the phage display system as well as filter binding assays or "real time" measuring of interaction using, for example, the BlAcore apparatus (Pharmacia); see references cited supra.

Furthermore, the present invention relates to the use of the polynucleotide, the vectors, peptides, polypeptides, antibodies and cells of the invention as well as compounds identified in accordance with a method of the invention described hereinabove for the preparation of a pharmaceutical composition for the treatment of diseases involving T-cell activation and associated with Th1 and Th2 immune response, for the treatment of acute and chronic rejection of allo-and xeno organ transplants and bone marrow transplantation, for the treatment of rheumatoid arthritis, lupus erythramatodes, multiple sklerosis, encephalitis, vasculitis, diabetes mellitus, pancreatitis, gastritis, thyroiditis, for the treatment of maligne disorders of T, B or NK cells, for the treatment of asthma, lepramatosis, Helicobacter pylori associated gastritis or for the treatment of skin tumors, adrenal tumors or lung tumors.

The polynucleotides, vectors, cells, proteins, (poly)peptides, antibodies, inhibitors, activators, pharmaceutical compositions, uses and methods of the invention can be used for the treatment of all kinds of diseases hitherto unknown as being related to or dependent on the modulation of T-cells. The pharmaceutical compositions, methods and uses of the present invention may be desirably employed in humans, although animal treatment is also encompassed by the methods and uses described herein.

These and other embodiments are disclosed and encompassed by the description and Examples of the present invention. Further literature concerning any one of the antibodies, methods, uses and compounds to be employed in accordance with the present invention may be retrieved from public libraries and databases, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http://www.tigr.org/, are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

### BRIEF DESCRIPTION OF DRAWINGS

This disclosure may best be understood in conjunction with the accompanying drawings, incorporated herein by references, which show:
- **Figure 1:**: Identification of TIRC7 from alloactivated T cells.
(A). Differential display identification of a 350 bp transcript upregulated 24 hours after alloactivation of human T lymphocytes. Each lane shows a mRNA expression pattern from a one-way MLR at either 0 or 24 h after activation. Two different MLRs (a and b) exhibited similar patterns of gene expression.
(B). Nucleotide sequence of TIRC7 cDNA.
The cDNA and deduced 614 amino acid sequences of the TIRC7 transcript is shown. Predicted transmembrane regions are underlined and bold.
- **Figure 2:**: Predicted secondary structure of TIRC7.
The predicted secondary structure of TIRC7 protein contains seven transmembrane spanning domains (TM). Peptides (P1 - P7) synthesized according to sequences in the putative intracellular amino terminus (NH₃), extracellular carboxy terminus (COOH), and the largest intracellular (IC) and extracellular (EC) loop were used to raise rabbit anti-TIRC7 polyclonal antibodies. Anti-TIRC7 antibodies with T cell response modulatory effect are given in the box.
- **Figure 3:**: TIRC7 is upregulated in T cell activation and is localized on the cell membrane.
(A). TIRC7 mRNA expression is upregulated in allostimulated human T cells. MLR-0 and MLR-24 indicate time points 0 h and 24 h, respectively, after coincubation of allogenic responder and stimulator lymphocytes.
(B). TIRC7 mRNA upregulation in MLR (24h) is prevented by Cyclosporin A (Cyc A).
- **Figure 4:**: Localization of TIRC7 protein.
(A). A single 75 kDa protein is detected by anti-TIRC7 antibodies (Ab79 is shown) predominantly in membrane protein extracts of human lymphocytes. Binding of Ab79 to TIRC7 is abolished in the presence of the respective peptide P2 (Ab+P2).
(B). The same single 75 kDa protein is also detected by an anti-*c-myc* antibody in membrane preparations of COS-7 cells transiently transfected with a c-*myc* tagged TIRC7 expression vector (COS7-t) but not in untransfected COS-7 cells (COS7-nt).
- **Figure 5:**: Anti-TIRC7 antibodies inhibit T cell proliferation and IL-2 production.
(A). Anti-TIRC7 antibodies (Ab73, Ab76, Ab79) directed against extracellularly located TIRC7 peptides (P4, P6, P2) inhibit proliferation in alloantigen stimulated T cells as determined by [³H]-thymidine incorporation. Inhibition was diminished when antibodies were preincubated with their respective peptides. Proliferation in a MLR after 24 h is displayed as positive control. Proliferation was not affected by preimmune serum (control Ab). Each bar represents mean and SD from seven independent experiments.
(B). Inhibition of proliferation by exogenous TIRC7 protein.
   In a one-way MLR *in vitro* translated TIRC7 protein inhibited proliferation in a dose-dependent manner. Protein dilutions are indicated. No inhibition of proliferation was observed when vector alone (vector) or an unrelated cDNA (unrel) was used in the *in vitro* translation preparation.
(C). Anti-TiRC7 antibodies inhibit Th1 specific cytokine expression. PHA stimulated human lymphocytes were coincubated with Ab73, Ab76 and Ab79, respectively. Supernatants of mitogen stimulated cultures were taken at 24 and 48 h. Cytokine expression in the supernatants were determined by ELISA. Each bar represents mean and SD from three independent experiments.
(D). Anti-TIRC7 antibody mediated inhibition of proliferation of PHA activated T cells is reconstituted by exogeneous IL-2. Each bar represents mean and SD from three independent experiments.
- **Figure 6:**: Anti-TIRC7 antibody targeting *in vivo* significantly prolongs allograft survival.
(A). Lewis rat recipients of Wistar Furth rat kidney allografts received either anti-TIRC7 Ab73 (n=7), control antibody from preimmune serum (n=7), or no treatment (n=7). Treatment was initiated at 2 h prior to and immediately after transplantation, and was repeated on day 1, 2, 4, and 6 post-transplantation. Animals treated with control antibody showed a mean survival time of 8 ± 1 days whereas mean survival time of animals representing the untreated control group was 7 ± 2 days. Six of the seven animals in the experimental group maintained functional grafts for more than 45 days. One rat in the anti-TIRC7 antibody treated experimental group had a survival time of 21 days. As assessed at day 45 after transplantation, the mean survival time in this group was 41,5 days (p< 0.001 vs controls).
- **Figure 7:**: Histological analysis of kidney allografts at day 7 post-transplantation.
(A). Kidney allografts of rats receiving control antibodies showed severe tissue destruction and diffuse mononuclear infiltration which was similar to histological findings in the kidney allografts of untreated animals.
(B). Renal allografts of two additional anti-TIRC7 antibody treated animals sacrificed at day 7 showed very mild interstitial infiltration of mononuclear cells. Tissue lessions were not identified in the allografts of these animals.

A better understanding of the present invention and of its many advantages will be had from the following examples, given by way of illustration.

### Examples

### Example 1: Cloning of a novel membrane protein (TIRC7) encoding a gene that is differentially expressed in alloactivated human T cells

To identify novel genes induced during the early stages of T cell activation in response to alloantigens, differential display RT-PCR analysis of mRNA expression was performed at time 0 and 24 h after initiation of a human mixed lymphocyte culture (MLR). In conformance with institutional policies regarding human experimentation, peripheral blood lymphocytes (PBLs) were isolated from healthy human volunteers using standard Ficoll centrifugation methods and diluted into RPMI containing 10% fetal calf serum. Responder PBLs were stimulated with equal numbers of irradiated (3000 rad, 13 min) stimulator PBLs. Cells were co-cultured for 24 h in tissue flasks at an initial concentration of 10⁶ cells/ml for RNA isolation. Total RNA was isolated from MLR at 0 and 24 h using the RNAzol B method (Tel-Test, Inc) and differential display was performed as described previously (Kojima et al., 1996). Briefly, 2 µg of total RNA was reverse transcribed using an oligo-dT primer and 200 U MMLV reverse transcriptase (Gibco/BRL). A 40 cycle PCR amplification with a total volume of 10 µl was performed by using 1 µg of cDNA, 1.25 mM MgCl₂, 50 mM KCl, 10 mM Tris-HCl (pH 8.3), 2.5 nM primer. 5 µCi ³⁵S-dATP, and 0.3 U *Taq* polymerase. The primers for the PCR amplification were: 5'- GACGGAACAGCTTC -3' (SEQ ID No. 10) and 5'-TGCGTCTGGTTCT-3' (SEQ ID No. 11). The PCR products were stored at 4°C and separated by electrophoresis in 6% polyacrylamide-urea gels, transferred to filter paper, dried, and autoradiographed. The differentially expressed cDNA fragment was excised from the gel. eluted, reamplifed, cloned into pBluescriptSK⁺ plasmid, and sequenced at the Howard Hughes Biopolymers Research Facility or the Dana Farber Cancer Institute Biopolymer Facility at Harvard Medical School. Homology searches were performed using BLAST at NCBI. Alignments were performed using Geneworks 2.1.1. As shown in Figure 1A, a 350 bp cDNA fragment was identified which was induced at 24 h after stimulation, and was used to identify several clones from λ-gt-10 cDNA libraries from human T cells. A λ gt-10 cDNA library (Clontech) prepared from human T cells activated for 48 hr with PHA was screened with the 350 bp TIRC7 cDNA fragment. Briefly, plaque lifts of 1,200,000 independent cDNA clones were hybridized with a ³²P-labeled cDNA for 24 hrs at 42°C in 40% formamide, 10% dextran sulfate, 4 X SSC (1 X SSC consists of 150 mM NaCl, 15 mM sodium citrate, pH 7.0), 0.8 X Denhardt's solution (1 X Denhardt's contains of 0.02% polyvinylpyrolidone, 0.02% Ficoll, 0.02% bovine serum albumin), 0.5% sodium dodecyl sulfate (SDS), and 20 mg salmon sperm DNA. The filters were washed twice for 20 min at room temperature with 2 X SSC, 10% SDS and for 30 min at 65°C with 0.2 X SSC, 10% SDS followed by autoradiography. Three positive clones were selected and plaque purified. cDNA was sequenced in both directions using a primer walking strategy. A PAC genomic library was screened using a 2 kb cDNA probe containing the ORF cDNA of TIRC7, and the entire genomic cDNA of TIRC7 and OC116 was bidirectinally sequenced. Sequence analysis revealed a 2488 bp cDNA (SEQ ID NO: 1) which was designated as TIRC7 (T cell immune response cDNA7; Gen Bank Accession Number: AF025374), containing an open reading frame of 1842 nt and predicting a protein length of 614 amino acids (SEQ ID No. 2) (Figure 1B). Furthermore, a second cDNA of TIRC7 was cloned (SEQ ID No. 12) encoding an amino acid sequence that is identical to that of the protein encoded by the other TIRC7 cDNA except for one amino acid substitution at amino acid position 121 (Arg → Gln). It was therefore concluded that both cDNAs represent alleles of the TIRC7 encoding gene. Hydrophobicity analysis of the protein sequence revealed seven hydrophobic domains, compatible with transmembrane spanning domains. The N-terminus of TIRC7 lacks a consensus signal peptide sequence and followed by seven hydrophobic domains predicting a topology of an intracellular N-terminus and extracellularly oriented C-domain (Figure 2). TIRC7 contains multiple putative sites of post-translational modification including phosphorylation sites for PKC (at amino acids 58, 98, and 148) and PKA (at amino acid 21), as well as N-linked glycosylation sites (at amino acid 267 and 287). No amino acid homology was found with any proteins known to be involved in T cell activation. TIRC7 does share amino acid homology (12-83%) with several proteins reported as putative subunits of the vacuolar proton pump H⁺-ATPase (VPP) in a variety of species (Bowman et al., 1988; Lee et al., 1990; Peng et al., 1994; Perin et al., 1991, Manolson et al., 1992, Manolson et al., 1994; Solioz and Davies., 1994; Li et al., 1996). Analysis of the complete genomic DNA organization of TIRC7 revealed that TIRC7 and a recently reported human osteoclast specific cDNA, named OC116 (Li et al., 1996), are alternatively spliced transcripts of the same gene. The function of OC116 is so far unknown and the 2640 nt mRNA, encoding a 814 residue protein, was demonstrated to be exclusively expressed in human osteoclast cells. The regions of strongest homology between TIRC7 and these putative VPPs are predominantly in their predicted transmembrane domains and the C-termini. Thus, TIRC7 belongs to a larger family of structurally related membrane proteins whose functions have not been clearly elucidated.

To determine the expression kinetics of TIRC7, Northern blot analysis of total RNA from alloantigen activated lymphocytes was performed. Northern blots were prepared with 7-10 µg of total RNA as described previously (Kojima et al., 1996). Poly(A)⁺ Northern blots containing RNA from various human tissues were purchased from Clontech. Northern blots were probed with the full-length TIRC7 cDNA or a TIRC7-specific cDNA fragment (nt 52-391 ). Overnight hybridizations were performed with ³²P labeled cDNA probes (10⁶ cpm/µl) at 42°C in 40% formamide, 10% dextran sulfate, 4 X SSC, 7 mM Tris (pH 7.6), 0.8 X Denhardt's solution, 0.02 mg/ml salmon sperm DNA, and 10% SDS. Blots were washed twice in 2 X SSC and 0.1 % SDS for 20 min at room temperature, once at 65°C in 0.2 X SSC, 0.1 % SDS and autoradiographed at -80°C. A TIRC7 specific cDNA probe detected the expected 2.5 kb transcript as well as an additional 4 kb mRNA of unknown origin (Figure 3A-B). Alloactivation of T cells resulted in a 20-fold upregulation of TIRC7 expression at 24 h (Figure 3A). TIRC7 expression was transient with no increase at 1 h, peak expression at 24 h, and a return to baseline at 72 h. To demonstrate that the upregulation of TIRC7 occurred in the responder T cell population, an additional MLR was performed using stimulators depleted of T cells. For studies on induction or inhibition of TIRC7 expression, PBLs were exposed to concanavalin A (10 ng/ml), phytohemagglutinin (PHA) (20 mg/ml), *Staphylococcus aureus* enterotoxin B (10 µg/ml), OKT3-mAb (10 mg/ml), cyclosporine A (1 mg/ml) or rIL-2 (10 U/ml). The stimulation with OKT3-mAb was carried out by immobilizing the antibody on plastic culture plates overnight at 4°C before adding the cell suspension. For RNA isolation from CD4⁺ and CD8⁺ human T cells PBLs were incubated with immunomagnetic beads coated with anti-CD4 or anti-CD8 IgG and then subjected to magnetic separation. TIRC7 mRNA expression was found to be increased in responder T cells 24 h after co-culture.

Cyclosporine A (Cyc A), an inhibitor of the calcineurin dependent T cell activation pathways, blocked the induction of TIRC7 in a MLR (Figure 3B). Furthermore, exogenous IL-2 was a potent inducer of TIRC7 expression, whereas a modest increase in TIRC7 expression was observed with *Staphylococcus aureus* enterotoxin B (SEB) or OKT3-mAb stimulation after 24 h, though OKT3-mAb increased TIRC7 expression after 48 h to similar levels induced by alloantigen. In contrast, neither concanavalin A (ConA) nor phytohemagglutinin (PHA) increased TIRC7 expression at 24 or 48 h.
Northern analysis revealed that TIRC7 is almost exclusively expressed in immune tissues and exhibits high levels of mRNA expression in spleen, lymph nodes, peripheral blood, and appendix, whereas lower levels of expression are observed in bone marrow, fetal liver and thymus, respectively. TIRC7 was also detected in CD4⁺ and CD8⁺ lymphocytes but not in EBV-transformed primary B cells, Burkitt's lymphoma cells, EBV-infected Burkitt's lymphoma cells, and resting or activated Jurkat cells.

In Western blot analysis, a single protein of approximately 75 kDa molecular mass was detected predominantly in membrane extracts of human lymphocytes (Figure 4A). PBLs were lysed in 50 mM Tris (pH 7.59), 150 mM NaCl, 1% triton, aprotinin (0.15 U/ml), 20 mM leupeptin, and 1 mM phenlymethylsulfonylfluoride. Lysates were separated by SDS-PAGE and transferred to a nitrocellulose membrane. The membranes were incubated with primary (see Example 2, infra) and secondary antibody for 1 h each, and bound primary antibody was detected by horseradish peroxidase-conjugated secondary antibody followed by enhanced chemiluminescence (Boehringer). Polyclonal Ab79 (see Example 2, infra) was used at 1:5000 dilution. The secondary antibody (anti-rabbit-IgG) conjugated with peroxidase was used at 1:2000 dilution. To detect the TIRC7/c-*myc* fusion protein the anti-c-*myc* antibody was used at 1:1000 dilution and the secondary antibody (anti-mouse-IgG) used at 1:2000 dilution. The same protein was found in membrane preparations from CHO cells and COS-7 cells (Figure 4B) stably and transiently transfected with a *c-myc* tagged TIRC7 expression vector, respectively. The full-length TIRC7 ORF was cloned upstream of a c-*myc* epitope sequence to create a fusion protein construct in a mammalian expression vector (Progmega). Transient transfection of COS7 cells and stable transfection of CHO cells was performed by lipofectamine transfection method as described (Schülein et al., 1996). An anti-c-*myc* antibody (InVitrogen) was used to detect the protein. TIRC7 localization to the cell membrane was confirmed by confocal microscopy and flow cytometric analysis in human lymphocytes as well as in stably transfected CHO cells.

### Example 2: TIRC7 mediates an essential signal during early events of T cell activation

The functional significance of a number of proteins required in T cell activation has been determined by modulation of their signaling by targeting with specific antibodies. To examine whether antibodies directed against TIRC7 could alter the T cell proliferative response. seven synthetic peptides representing different domains of the TIRC7 protein (P1-P7. 12mers; SEQ ID Nos. 3 to 9) were used to produce polyclonal rabbit antipeptide antibodies (Figure 2A). Antigenic non-transmembrane regions of TIRC7 were identified using PSORT and PC-GENE and used to design short peptide sequences. Purified synthetic peptides (P1-P7) (Laboratories of Henklein, Berlin, Germany) were used for immunization of rabbits (Seramun, Berlin, Germany). Animals were boosted after three and six weeks. A total of 14 polyclonal antibodies were prepared against 7 different peptides (P1-P7). The pooled antisera were purified by affinity chromatography after binding of peptide to BSA. All antibodies were tested by ELISA with their respective peptides. As shown in Figure 5A, three of the antibodies Ab73, Ab76 and Ab79, which were directed against the extracellularly located domains P4, P6, and P2 (SEQ ID Nos. 6, 8 and 2), respectively, were found to inhibit the proliferation of alloactivated T cells by 87-93%. For proliferation assays, responder PBLs were plated in the presence of an equal number of irradiated-stimulator cells (total of 2 x 10 ⁶ cells / ml) with either media alone, antibodies or control serum into each well of a round-bottomed 96-well microtiter plate in a final culture volume of 200 µl. Anti-TIRC7 anti-sera were added in 1:500 dilutions to MLR. The plates were incubated at 37°C, 5% CO₂ and pulsed for the final 18 h of the culture with 1 µCi [³H]-thymidine (ICN Biochemicals) per well. All plates were harvested and counted on a Betaplate liquid scintillation counter. Counts were represented as the mean cpm of quadruplicate wells harvested at 72 h following the 18 hr pulse. To prevent complement lysis of the cells, antibodies were incubated at 52°C for 20 min prior to use. Peptide blocking experiments were performed by coincubation of the antibody with the appropriate peptide for 30 min at 4°C prior to initiating the MLR_{G}. For studies using TIRC7 protein, serial dilutions (1:200, 1:300, 1:600) of the dialyzed *in vitro* translated TIRC7 protein were added to MLRs and inhibition was measured by ³H-thymidine incorporation. The cytokine expression for IL-2, IL-4 and interferon-γ were detected in culture supernatants by ELISA. Commercial kits were used for IL-2 (Laboserv), IL-4 (Laboserv) and IFN-γ (Medgenix). The anti-TIRC7 antibodies inhibited T cell proliferation in a dose dependent manner. Inhibition was diminished when the respective peptides were added to the reaction to specifically neutralize the antibody. The antibodies had no effect when added 24 h - 72 h after initiating the MLR, indicating that the TIRC7 mediated signal was specific for an early event in the T cell response. These three anti-TIRC7 antibodies also caused efficient inhibition of T-cell activation induced by ConA, PHA, and OKT3-mAb, respectively.

Membrane proteins associated with T cell activation are often involved in ligandreceptor interactions that can be blocked by exogenous soluble protein, as has been demonstrated by blocking of CD28/B7 interaction with the soluble protein CTLA4Ig (Linsley et al., 1992). *In vitro* translated TIRC7 protein was therefore tested for its ability to inhibit the MLR by adding it to MLR cultures at time 0 at dilutions of 1:200, 1:300, 1:600. 2 µg of TIRC7 cDNA were translated in an *in vitro* translation TNT lysate system (Promega) containing ³⁵S methionine (ICN). The product was visualized by SDS-page (11 %) and autoradiography. For MLR inhibition experiments, TIRC7 protein was synthesized *in vitro* in the presence of microsomal membranes. The *in vitro* translation mixture was then suspended in 500 µl PBS and dialyzed against PBS for 24 h. As shown in Figure 5B, exogenous TIRC7 protein significantly suppressed the proliferation of alloreactive T cells in a dose-dependent manner. In control experiments, no inhibition was seen using either vector alone (Figure 5B) or another unrelated cDNA in the *in vitro* translation reaction mixture.

### Example 3: Targeting of the TIRC7 mediated signal inhibits Th1 specific cytokine expression, which is reversed by exogenous rIL-2

To further differentiate whether signals mediated by TIRC7 differentially affect T cell subsets, human T cells were challenged with either OKT3-mAb, ConA or PHA, and the cytokine profiles specific for Th1 and Th2 lymphocyte subsets were analyzed in the presence and absence of anti-TIRC7 antibodies. As shown in Figure 5C, a significant decrease of the Th1-specific cytokines IL-2 and IFN-γ was observed at 48 h in all cultures of PHA stimulated lymphocytes. Of the three mitogens tested, the IL-2 and IFN-γ downregulation occurred in ConA and PHA activated cultures at 24 h, whereas the OKT3-mAb stimulated T cells showed a significant decrease of IL-2 at 24 h but of IFN-γ only after 48 h. No downregulation of IL-4 production, which is specific for Th2 lymphocytes, was observed in any of the mitogen activated T cells after 24 and 48 h. Remarkably, exogenous addition of recombinant IL-2 to mitogen-activated cultures incubated with anti-TIRC7 antibodies reconstituted the diminished T cell proliferation (Figure 5D).

### Example 4: TIRC7 antibody targeting significantly prolongs renal allograft survival in vivo

The effect of modulating the TIRC7 mediated signal was studied in an animal model featuring kidney transplantation from Wistar Furth to Lewis rats. Male inbred rats 200-250 g (Harlan Winkelmann, Germany) were used throughout the experiment. Wistar Furth rats (WF, RT1^{u}) were grafted into bilaterally nephrectomized Lewis rats (LEW, RT1¹) using microsurgical techniques; ischemic time was 30 ± 5 min. Cryostat sections were fixed in formalin. The fixed tissue was paraffin embedded, and tissue sections were stained with hematoxylin and eosin. In initial experiments, anti-human TIRC7 antibodies were tested for their ability to inhibit the proliferation of Lewis rat lymphocytes stimulated with irradiated Wistar Furth rat lymphocytes *in vitro*. Ab73 was shown to profoundly block rat T cell proliferation. In kidney transplant experiments, animals remained either untreated (n=7), received preimmune rabbit serum (n=7) or were treated with anti-TIRC7 antibody Ab73 (n=7), 2 h before, directly after and on days 1, 2, 4 and 6 after transplantation. No side effects except for transient mild diarrhea were observed in the anti-TIRC7 antibody treated group. Anti-TIRC7 antibody significantly prolonged the graft survival time of treated animals (p < 0, 001). Six of seven allografts of the anti-TIRC7 treated animals remained functional for more than 45 days after completion of antibody administration. One animal treated with anti-TIRC7 antibody died at day 21. In contrast, all animals in both control groups died of renal failure by day 7 to 9 after transplantation (Figure 6). Histological examination of kidney grafts from two additional antibody treated animals sacrificed at day 7 post-transplantation demonstrated very mild lymphocytic infiltration but no signs of tissue necrosis (Figure 7B). In contrast, Kidney grafts from control animals displayed remarkable evidence of acute graft rejection including diffuse mononuclear cell infiltrates as well as extensive areas of necrosis (Figure 7A).

### Summary: TIRC7 is a novel T-cell accessory protein

TIRC7 represents a novel protein that plays an essential role in T cell activation. Early after stimulation of the T cell receptor the level of TIRC7 mRNA is transiently increased. This increase in expression appears to rely on an IL-2 dependent pathway, as upregulation of TIRC7 mRNA is also observed after incubation of T cells with IL-2 and blockade of TIRC7 upregulation is achieved with cyclosporine A. T cell stimulation by mitogens such as ConA or PHA consistently fail to upregulate TIRC7 expression. The pattern of tissue expression suggests that TIRC7 is a product of mature lymphoid cells, as TIRC7 is expressed in all lymphoid tissues with low expression only in thymus, bone marrow and fetal liver. The TIRC7 protein is predominantly expressed on the cell membrane, consistent with a target for an external ligand. The seven transmembrane domain structure predicts three extracellular loops and an extracellularly oriented carboxy terminus.
Anti-TIRC7 antibodies directed against the extracellular domains, but not those recognizing predicted intracellular domains of the protein, are able to efficiently suppress the proliferation of T cells in response to alloactivation in a mixed lymphocyte culture or in response to mitogens. The inhibitory effect of anti-TIRC7 antibodies on T cells induced by a variety of different stimulatory pathways suggests that TIRC7 plays a central role in T cell activation. Moreover, inhibition of T cell proliferation in MLR by antibody targeting of TIRC7 suggest the existence of a ligand specifically interacting with TIRC7. Support for this hypothesis is provided by the dose-dependent inhibition of T cell proliferation in a MLR in the presence of soluble *in vitro* translated TIRC7 protein. TIRC7 shares 38% amino acid homology with J6B7, a protein isolated from a mouse T cell line (Lee et al., 1990). Like TIRC7, J6B7 exhibits considerable homology to the putative rat H⁺-ATPase subunit VPP116 (Manolson et al., 1992). *In vitro* translated J6B7 protein was demonstrated to inhibit mouse T cell proliferation in a MLR by 89%, which is comparable with the results obtained with *in vitro* translated soluble TIRC7 protein in human MLR in the present study.
Antibody targeting of TIRC7 has a selective inhibitory effect on the Th1 lymphocyte subset, as evidenced by the inhibition of IL-2 and IFN-γ, but not IL-4, cytokine production. With anti-TIRC7 antibody treatment the cells appear to remain in an unresponsive, but functional, state since exogenous recombinant IL-2 reversed the antiproliferative effect of the anti-TIRC7 antibodies. The ability of an anti-TIRC7 antibody to prevent allograft rejection in the *in vivo* model of rat kidney transplantation reflects the findings obtained in the *in vitro* studies. Moreover, the effects of antibody targeting of TIRC7 are quite similar to those observed by targeting of costimulatory molecules. TIRC7 does not share structural or sequence homology with any of the known T cell accessory molecules. Thus, TIRC7 may participitate in a distinct signaling pathway induced early in the course of T cell activation.

Given the functional similarities between TIRC7 and the known T cell accessory molecules, it is expected that the structural novelty of TIRC7 will contribute to the understanding of distinct mechanisms in the T cell response. Moreover, the striking capacity of anti-TIRC7 antibody to significantly prolong allograft survival *in vivo* provide a novel approach for a selective inhibition of undesired T cell activation in human organ transplantation and autoimmune diseases.

Those skilled in the art will recognize, or be able to ascertain using no more than routine experimentation, many equivalents to the specific embodiments of the invention described herein. Such equivalents are intended to be encompassed by the following claims.

### References

Abbas, Nature 383 (1996), 787-793

Bachmann, Immunity 7 (1997), 549-557

Blazar J. Immunol. 157 (1996), 3250-3259

Bluestone, Immunity 2 (1995), 555-559

Bowman, J. Biol. Chem. 263 (1988), 13994-14001

Cosimi, Transplantation 32 (1981), 535-539

Crabtree, Science 243 (1989), 355-361

Kirk, Proc. Natl. Acad. Sci. USA 94 (1997), 8789-8794

Kojima, J. Biol. Chem. 271 (1996), 12327-12332

Larsen, Nature 381 (1996), 434-438

Lazarovits, Nature 380 (1996), 717-720

Lee, Mol. Immunol. 27 (1990), 1137-1144

Lenschow, Annu. Rev. Immunol. 14 (1996), 233-58

Lenschow, Science 257 (1992), 789-792

Linsley, Annu. Rev. Immunol. 11 (1993), 191-212

Linsley, Science 257 (1992), 792-95

Li, Biochem. Biophy. Res. Commun. 218 (1996), 813-821

Manolson, J. Exp. Biol, 172 (1992), 105-112

Manolson,. Biol. Chem. 269 (1994) J, 14064-14074

Mohammed, J. Exp. Med. 181 (1995), 1869-1874

Peng, J. Biol. Chem. 269 (1994), 17262-17266

Perez, Immunity 6 (1997), 411-417

Perin, J. Biol. Chem. 266 (1991), 3877-3881

Schreiber, Immunology Today 13 (1992), 136-42

Schülein, J. Biol. Chem. 271 (1996), 28844-28852

Schwartz, Cell 71 (1992), 1065-1068

Solioz, J. Biol. Chem. 269 (1994), 9453-9459

Turka, Proc. Natl. Acad. Sci. USA 89 (1992), 11102-11105

Walunas, Immunity 1 (1994), 405-413

Xu, Immunity 1 (1994), 423-431

## Claims

1. A polynucleotide encoding a TIRC7 membrane protein of approximately 75kDa or a fragment thereof capable of inhibiting the proliferation of T-cells in response to alloactivation in a mixed lymphocyte culture or in response to mitogens, and comprising a DNA sequence selected from the group consisting of
(i) DNA sequences comprising a nucleotide sequence encoding the amino acid sequence depicted in SEQ ID NO. 2 or SEQ ID NO. 13 from amino acid position 1 to 614 or from amino acid position 1 to 601;
(ii) DNA sequences comprising the nucleotide sequence depicted in SEQ ID NO. 1 or SEQ ID NO. 12;
(iii)DNA sequences comprising a nucleotide sequence encoding a fragment of the protein encoded by the DNA sequence of (i) or (ii), wherein said fragment is capable of inhibiting the proliferation of T-cells in response to alloactivation in a mixed lymphocyte culture or in response to mitogens; and
(iv)DNA sequences the complementary strand of which hybridizes under stringent hybridization conditions with, and which are at least 70% identical to the polynucleotide as defined in any one of (i) to (iii).

2. Use of a nucleic acid molecule of at least 15 nucleotides in length hybridizing specifically with a polynucleotide of claim 1 or with a complementary strand thereof for the detection and/or amplification of a polynucleotide of claim 1 or for the repression of expression of a gene comprising the polynucleotide of claim 1, in vitro.

3. A vector comprising the polynucleotide of claim 1.

4. The vector of claim 3, wherein said polynucleotide is operably linked to regulatory sequences allowing the transcription and optionally expression of said nucleic acid molecule.

5. A host cell comprising a polynucleotide of claim 1 under the control of a heterologous promoter or the vector of claim 3 or 4.

6. A method for the production of a TIRC7 membrane protein or a biologically active fragment thereof comprising:
(a) culturing the host cell of claim 5 under conditions allowing for the expression of the protein; or
(b) in vitro translation of the polynucleotide of claim 1;
and recovering the protein produced in (a) or (b).

7. A TIRC7 membrane protein or a fragment thereof capable of inhibiting the proliferation of T-cells in response to alloactivation in a mixed lymphocyte culture or in response to mitogens, and encoded by the polynucleotide of claim 1 or produced by the method of claim 6.

8. An antibody specifically recognizing an extracellular domain of the TIRC7 membrane protein of claim 7.

9. A cell that has been modified to express the antibody of claim 8.

10. A pharmaceutical composition comprising a peptide or polypeptide being capable of inhibiting T-cell stimulation through the TIRC7 membrane protein as defined in claim 7 and/or being recognized by an antibody capable of inhibiting T-cell stimulation through the TIRC7 membrane protein encoded by a fragment of the polynucleotide of claim 1 or an antibody specifically recognizing said peptide or polypeptide, and optionally a pharmaceutically acceptable carrier.

11. The pharmaceutical composition of claim 10, wherein said peptide or polypeptide is in a soluble form.

12. The pharmaceutical composition of claim 10 or 11, wherein said peptide or polypeptide comprises the amino acid sequence depicted in any one of SEQ ID NOS: 3 to 9.

13. The pharmaceutical composition of any one of claims 10 to 12 further comprising a second agent which inhibits T-cell stimulation.

14. The pharmaceutical composition of claim 13, wherein the second agent is an antibody to a receptor or ligand involved in T-cell stimulation or is a soluble form of said receptor.

15. The pharmaceutical composition of claim 14, wherein said second agent blocks interaction of the CD2, CD28, CTLA4 or CD40 surface receptor with a CD2, CD28, CTLA4 or CD40 ligand.

16. The pharmaceutical composition of any one of claims 10 to 15 for use in cell or organ transplantation, for the treatment of autoimmune, allergic or infectious diseases, or for the treatment of tumors.

17. A diagnostic composition comprising a polynucleotide of claim 1, the vector of claim 3 or 4, the cell of claim 5 or 9, the protein of claim 7, or the antibody of claim 8 or a (poly)peptide or antibody as defined in any one of claims 10 to 12; and optionally suitable means for detection.

18. An in vitro method for inducing or maintaining unresponsiveness of a T-cell to an antigen comprising contacting the T-cell with an agent which inhibits stimulation of the T-cell through the TIRC7 membrane protein of claim 7.

19. Use of an agent which inhibits T-cell stimulation through the TIRC7 membrane protein of claim 7 for the preparation of a pharmaceutical composition for inducing or maintaining T-cell unresponsiveness to an antigen in a subject.

20. The method of claim 18 or the use of claim 19, wherein the agent blocks an interaction of the TIRC7 membrane protein with its ligand.

21. The method or the use of claim 20, wherein the agent is a peptide or polypeptide being capable of inhibiting T-cell stimulation through the TIRC7 membrane protein and/or being recognized by an antibody capable of inhibiting T-cell stimulation through the TIRC7 membrane protein encoded by a fragment of the polynucleotide of claim 1 or an antibody specifically recognizing said peptide or polypeptide.

22. The method of any one of claims 18, 20 or 21 or the use of any one of claims 19 to 21, further comprising the use of a second agent as defined in any one of claims 13 to 16.

23. A pharmaceutical composition comprising a first agent which stimulates a T-cell through the TIRC7 membrane protein of claim 7 and optionally a pharmaceutically acceptable carrier.

24. The pharmaceutical composition of claim 23, wherein said agent is a ligand of the TIRC7 membrane protein or is at least one anti-TIRC7 membrane protein antibody.

25. The pharmaceutical composition of claim 23 to 24 further comprising a second agent which stimulates T-cell proliferation.

26. The pharmaceutical composition of claim 25, wherein said agent is IL-2, IL-4 or an agent which stimulates a T-cell through a CD2, CD28, CD40 or CTLA4 surface receptor.

27. An in vitro method for restoring responsiveness to an antigen by a T-cell which is unresponsive to the antigen, comprising contacting the T-cell in the presence of the antigen with a first agent which stimulates the T-cell through the TIRC7 membrane protein of claim 7.

28. The method of claim 27, wherein the TIRC7 ligand is expressed on a cell surface by introducing to the cell a nucleic acid molecule encoding the TIRC7 ligand in a suitable form for expression of the TIRC7 ligand on the cell surface.

29. The method of claim 28, wherein the cell is a tumor cell.

30. The method of any one of claims 27 to 29, further comprising contacting the T-cell with a second agent as defined in claim 25 or 26.

31. The method of claim 30, wherein the T-cell is contacted with the second agent prior to being contacted with the first agent.

32. The method of claim 30 or 31, wherein a CD2, CD28, CD40 or CTLA4 ligand is expressed on the cell surface by introducing into the cell a nucleic acid molecule encoding the CD2, CD28, CD40 or CTLA4 ligand in a form suitable for expression of said ligand on the cell surface.

33. Use of a first agent as defined in claim 23 or 24 for the preparation of a pharmaceutical composition for stimulating a T-cell response to a tumor cell in a subject with a tumor.

34. The use of claim 33 wherein the tumor cell is modified to express a TIRC7 ligand and/or a CD2, CD28, CD40 or CTLA4 ligand preferably on the tumor cell surface.

35. The use of claim 33 or 34, wherein the tumor cell is a modified tumor cell from the subject.

36. The use of any one of claims 19 to 22 or 33 to 35, wherein the subject is a recipient of peripheral stem cells or bone marrow transplant.

37. The use of claim 36, wherein the pharmaceutical composition is designed for contacting with peripheral stem cells or bone marrow cells prior to transplantation into the recipient.

38. A method for identifying T-cell activating or co-stimulating compounds or for identifying inhibitors of T-cell activation and stimulation comprising
(a) culturing T-cells in the presence of the protein of claim 7, the antibody of claim 8, the cell of claim 5 or 9 or a peptide or polypeptide being capable of inhibiting T-cell stimulation through the TIRC7 membrane protein and/or being recognized by an antibody capable of inhibiting T-cell stimulation through the TIRC7 membrane protein encoded by a fragment of the polynucleotide of claim 1 or an antibody specifically recognizing said peptide or polypeptide and, optionally, in the presence of a component capable of providing a detectable signal in response to T-cell activation, with a compound to be screened under conditions permitting interaction of the compound with the (poly)peptide, antibody or cell(s); and
(b) detecting the presence or absence of a signal generated from the interaction of the compound with the cells.

39. Use of peptide or polypeptide being capable of inhibiting T-cell stimulation through the TIRC7 membrane protein and/or being recognized by an antibody capable of inhibiting T-cell stimulation through the TIRC7 membrane protein encoded by a fragment of the polynucleotide of claim 1 or an antibody specifically recognizing said peptide or polypeptide, the polynucleotide of claim 1, the vector of claim 3 or 4, the protein of claim 7, the antibody of claim 8, the cell of claim 5 or 9 or the compound indentified according to the method of claim 38 for the preparation of a pharmaceutical composition for the treatment of acute and chronic diseases, involving T-cell activation and Th1 and Th2 immune response, for the treatment of acute and chronic rejection of allo-and xeno organ transplants and bone marrow transplantation, for the treatment of rheumatoid arthritis, lupus erythramatodes, multiple sklerosis, encephalitis, vasculitis, diabetes mellitus, pancreatitis, gastritis, thyroiditis, for the treatment of maligne disorders of T, B or NK cells, for the treatment of asthma, lepramatosis, Helicobacter pylori associated gastritis or for the treatment of skin tumors, adrenal tumors or lung tumors.

## Patentansprüche

1. Polynucleotid, das ein TIRC7 Membranprotein von ungefähr 75kDa oder ein Fragment davon codiert, das in der Lage ist die Proliferation von T-Zellen als Reaktion auf eine Alloaktivierung in einer gemischten Lymphozytenkultur oder als Reaktion auf Mitogene zu inhibieren, und eine DNA Sequenz enthält, ausgewählt aus der Gruppe bestehend aus
(i) DNA Sequenzen, die eine Nucleotidsequenz enthalten, welche die in SEQ ID NO. 2 oder SEQ ID NO. 13 dargestellten Aminosäuresequenzen von Aminosäureposition 1 bis 614 oder von Aminosäureposition 1 bis 601 codieren;
(ii) DNA Sequenzen, die eine in SEQ ID NO. 1 oder SEQ ID NO. 12 dargestellte Nucleotidsequenz enthalten;
(iii) DNA Sequenzen, die eine Nucleotidsequenz enthalten, welche ein Fragment des Proteins codieren, das durch eine DNA Sequenz von (i) oder (ii) codiert wird, wobei das Fragment in der Lage ist, die Proliferation von T-Zellen als Reaktion auf Alloaktivierung in einer gemischeten Lymphozytenkultur oder als Reaktion auf Mitogene zu inhibieren; und
(iv) DNA Sequenzen, deren komplementärer Strang unter stringenten Bedingungen mit einem der in (i) bis (iii) definierten Polynucleotide hybridisiert, und die mindestens zu 70% mit einem dieser Polynucleotide identisch sind.

2. Verwendung eines Nukleinsäuremoleküls von mindestens 15 Nucleotiden Länge, das spezifisch mit einem Polynucleotid nach Anspruch 1 oder mit einem dazu komplementären Strang hybridisiert zum Nachweis und/oder der Amplifikation eines Polynucleotids nach Anspruch 1 oder zur Repression der Expression eines Gens, das das Polynucleotid nach Anspruch 1 enthält, in vitro.

3. Vektor enthaltend das Polynucleotid nach Anspruch 1.

4. Der Vektor nach Anspruch 3, wobei das Polynucleotid funktionell mit regulatorischen Sequenzen verknüpft ist, die die Transkription und gegebenenfalls Expression des Nukleinsäuremoleküls ermöglichen.

5. Wirtszelle, die ein Polynucleotid nach Anspruch 1 unter der Kontrolle eines heterologen Promotors oder den Vektor nach Anspruch 3 oder 4 enthält.

6. Verfahren zur Herstellung eines TIRC7 Membranproteins oder eines biologisch aktiven Fragmentes davon, umfassend:
(a) Kultivieren der Wirtszelle nach Anspruch 5 unter Bedingungen, die die Expression des Proteins ermöglichen; oder
(b) in vitro Translation des Polynucleotids nach Anspruch 1;
und Gewinnung des in (a) oder (b) hergestellten Proteins.

7. TIRC7 Membranprotein oder ein Fragment davon, das in der Lage ist die Proliferation von T-Zellen als Reaktion auf Alloaktivierung in einer gemischten Lymphozytenkultur oder als Reaktion auf Mitogene zu inhibieren, und das durch das Polynucleotid nach Anspruch 1 codiert oder durch das Verfahren nach Anspruch 6 hergestellt ist.

8. Antikörper, der spezifisch eine extrazellulare Domäne des TIRC7 Membranproteins nach Anspruch 7 erkennt.

9. Zelle, die modifiziert wurde um den Antikörper nach Anspruch 8 zu exprimieren.

10. Pharmazeutische Zusammensetzung, umfassend ein Peptid oder Polypeptid, das in der Lage ist die Stimulation von T-Zellen durch das TIRC7 Membranprotein, wie in Anspruch 7 definiert, zu inhibieren und/oder von einem Antikörper erkannt wird, der in der Lage ist die Stimulation von T-Zellen durch das TIRC7 Membranprotein, das durch ein Fragment des Polynucleotids nach Anspruch 1 codiert wird, zu inhibieren, oder enthaltend einen Antikörper, der spezifisch das Peptid oder Polypeptid erkennt; und gegebenenfalls einen pharmazeutisch verträglichen Träger.

11. Pharmazeutische Zusammensetzung nach Anspruch 10, wobei das Peptid oder Polypeptid in einer löslichen Form vorliegt.

12. Pharmazeutische Zusammensetzung nach Anspruch 10 oder 11, wobei das Peptid oder Polypeptid die in einer der SEQ ID NOS: 3 bis 9 dargestellte Aminosäuresequenz umfasst.

13. Pharmazeutische Zusammensetzung nach einem der Ansprüche 10 bis 12, außerdem enthaltend ein zweites Agens, das die Stimulation von T-Zellen inhibiert.

14. Pharmazeutische Zusammensetzung nach Anspruch 13, wobei das zweite Agens ein Antikörper für einen an der T-Zell Stimulation beteiligten Rezeptor oder Liganden oder eine lösliche Form des Rezeptors ist.

15. Pharmazeutische Zusammensetzung nach Anspruch 14, wobei das zweite Agens die Interaktion des CD2, CD28, CTLA4 oder CD40 Oberflächenrezeptors mit einem CD2, CD28, CTLA4 oder CD40 Liganden blockiert.

16. Pharmazeutische Zusammensetzung nach einem der Ansprüche 10 bis 15 zur Verwendung in Zell- oder Organtransplantation, zur Behandlung von Autoimmun-, allergischen oder infektiösen Krankheiten, oder zur Behandlung von Tumoren.

17. Diagnostische Zusammensetzung, enthaltend ein Polynucleotid nach Anspruch 1, den Vektor nach Anspruch 3 oder 4, die Zelle nach Anspruch 5 oder 9, das Protein nach Anspruch 7 oder den Antikörper nach Anspruch 8 oder ein (Poly)Peptid oder einen Antikörper, wie in einem der Ansprüche 10 bis 12 definiert; und gegebenenfalls geeignete Mittel für den Nachweis.

18. In vitro Verfahren zur Induktion oder zum Aufrechterhalten der Unempfindlichkeit einer T-Zelle für ein Antigen, die das in Kontakt bringen einer T-Zelle mit einem Agens umfasst, das die Stimulation der T-Zelle durch das TIRC7 Membranprotein nach Anspruch 7 inhibiert.

19. Verwendung eines Agens, das die T-Zell-Stimulation durch das TIRC7 Membranprotein nach Anspruch 7 inhibiert zur Herstellung einer pharmazeutischen Zusammensetzung zur Induktion oder Aufrechterhaltung der T-Zell-Unempfindlichkeit für ein Antigen in einem Subjekt.

20. Verfahren nach Anspruch 18 oder Verwendung nach Anspruch 19, wobei das Agens eine Interaktion des TIRC7 Membranproteins mit seinem Liganden blockiert.

21. Verfahren oder Verwendung nach Anspruch 20, wobei das Agens ein Peptid oder Polypeptid ist, das in der Lage ist, die T-Zell-Stimulation durch das TIRC7 Membranprotein zu inhibieren und/oder von einem Antikörper erkannt wird, der in der Lage ist, die T-Zell-Stimulation durch das TIRC7 Membranprotein, welches durch ein Fragment des Polynucleotids nach Anspruch 1 codiert wird, zu inhibieren, oder ein Antikörper ist, der spezifisch das Peptid oder Polypeptid erkennt.

22. Verfahren nach einem der Ansprüche 18, 20 oder 21 oder Verwendung nach einem der Ansprüche 19 bis 21, außerdem umfassend die Verwendung eines zweiten Agens, wie in einem der Ansprüche 13 bis 16 definiert.

23. Pharmazeutische Zusammensetzung, enthaltend ein erstes Agens, das eine T-Zelle über das TIRC7 Membranprotein nach Anspruch 7 stimuliert, und gegebenenfalls ein pharmazeutisch verträglichen Träger.

24. Pharmazeutische Zusammensetzung nach Anspruch 23, wobei das Agens ein Ligand des TIRC7 Membranproteins oder mindestens ein anti-TIRC7 Membranprotein Antikörper ist.

25. Pharmazeutische Zusammensetzung nach Anspruch 23 bis 24, außerdem umfassend ein zweites Agens, das die T-Zell Proliferation stimuliert.

26. Pharmazeutische Zusammensetzung nach Anspruch 25, wobei das Agens IL-2, IL-4 oder ein Agens ist, das eine T-Zelle durch einen CD2, CD28, CD40 oder CTLA4 Oberflächenrezeptor stimuliert.

27. In vitro Verfahren zur Wiederherstellung der Empfindlichkeit für ein Antigen durch eine T-Zelle, die unempfindlich für das Antigen ist, umfassend das Inkontaktbringen der T-Zelle in der Anwesenheit des Antigens mit einem ersten Agens, das die T-Zelle über das TIRC7 Membranprotein nach Anspruch 7 stimuliert.

28. Verfahren nach Anspruch 27, wobei der TIRC7 Ligand durch Einführen in die Zelle eines Nukleinsäuremoleküls, das den TIRC7 Liganden in einer für die Expression des TIRC7 Liganden an der Zelloberfläche geeigneten Form codiert auf einer Zelloberfläche exprimiert wird.

29. Verfahren nach Anspruch 28, wobei die Zelle eine Tumorzelle ist.

30. Verfahren nach einem der Ansprüche 27 bis 29, außerdem umfassend das Inkontaktbringen der T-Zelle mit einem zweiten Agens, wie in Anspruch 25 oder 26 definiert.

31. Verfahren nach Anspruch 30, wobei die T-Zelle mit dem zweiten Agens in Kontakt gebracht wird bevor sie mit dem ersten Agens in Kontakt gebracht wird.

32. Verfahren nach Anspruch 30 oder 31, wobei ein CD2, CD28, CD40 oder CTLA4 Ligand durch Einführen eines Nukleinsäuremoleküls in die Zelle, das den CD2, CD28, CD40 oder CTLA4 Liganden in einer für die Expression des Liganden auf der Zelloberfläche geeigneten Form codiert, auf der Zelloberfläche exprimiert wird.

33. Verwendung eines ersten Agens, wie in Anspruch 23 oder 24 definiert, zur Herstellung einer pharmazeutische Zusammensetzung zur Stimulation einer T-Zell-Antwort auf eine Tumorzelle in einem Subjekt mit einem Tumor.

34. Verwendung nach Anspruch 33, wobei die Tumorzelle so modifiziert ist, dass sie einen TIRC7 Liganden und/oder einen CD2, CD28, CD40 oder CTLA4 Liganden exprimiert, vorzugsweise auf der Tumorzelloberfläche.

35. Verwendung nach Anspruch 33 oder 34, wobei die Tumorzelle eine modifizierte Tumorzelle des Subjekts ist.

36. Verwendung nach einem der Ansprüche 19 bis 22 oder 33 bis 35, wobei das Subjekt ein Empfänger von peripheren Stammzellen oder eines Knochenmarkstransplantats ist.

37. Verwendung nach Anspruch 36, wobei die pharmazeutische Zusammensetzung dafür bestimmt ist vor der Transplantation in den Empfänger mit peripheren Stammzellen oder Knochenmarkszellen in Kontakt gebracht zu werden.

38. Verfahren zur Identifizierung von T-Zell-aktivierenden oder co-stimulierenden Verbindungen oder zur Identifizierung von Inhibitoren der T-Zell-Aktivierung und Stimulierung umfassend
(a) das Kultivieren von T-Zellen in der Gegenwart des Proteins nach Anspruch 7, des Antikörpers nach Anspruch 8, der Zelle nach Anspruch 5 oder 9 oder eines Peptids oder Polypeptids, das in der Lage ist, die T-Zell-Stimulation durch das TIRC7 Membranprotein zu inhibieren und/oder das durch einen Antikörper, der in der Lage ist, die T-Zell-Stimulation durch das TIRC7 Membranprotein, das durch ein Fragment des Polynucleotid nach Anspruch 1 codiert wird, zu inhibieren oder eines Antikörpers, der spezifisch das Peptid oder Polypeptid erkennt, und gegebenenfalls, in der Gegenwart einer Komponente, die in der Lage ist ein nachweisbares Signal als Reaktion auf die T-Zell-Aktivierung zu liefern, mit einer zu überprüfenden Verbindung unter Bedingungen, die die Interaktion der Verbindung mit dem (Poly)Peptid, Antikörper oder Zelle(n) erlaubt; und
(b) das Nachweisen der Anwesenheit oder Abwesenheit eines Signals, das durch die Interaktion der Verbindung mit den Zellen erzeugt wird.

39. Verwendung eines Peptids oder Polypeptids, das in der Lage ist die T-Zell-Stimulation durch das TIRC7 Membranprotein zu inhibieren und/oder das durch einen Antikörper erkannt wird, der in der Lage ist, die T-Zell-Stimulation durch das TIRC7 Membranprotein, das durch ein Fragment des Polynucleotid nach Anspruch 1 codiert wird, zu inhibieren oder eines Antikörper, der spezifisch das Peptid oder Polypeptid erkennt, des Polynucleotids nach Anspruch 1, des Vektors nach Anspruch 3 oder 4, des Proteins nach Anspruch 7, des Antikörpers nach Anspruch 8, der Zelle nach Anspruch 5 oder 9 oder der Verbindung, die durch ein Verfahren nach Anspruch 38 identifiziert wurde für die Herstellung einer pharmazeutischen Zusammensetzung zur Behandlung akuter und chronischer Erkrankungen, die mit einer T-Zell-Aktivierung und Th1 und Th2 Immunantwort verbunden sind, zur Behandlung von akuten und chronischen Abstoßungsreaktionen nach Allo- und Xeno- Organtransplantaten und Knochenmarkstransplantationen, zur Behandlung von rheumatischer Arthritis, Lupus Erythramatodes, Multipler Sklerose, Encephalitis, Vasculitis, Diabetes Mellitus, Pancreatitis, Gastritis, Thyroiditis, zur Behandlung von bösartigen Erkrankungen von T, B oder NK Zellen, zur Behandlung von Asthma, Lepramatosis, Helicobacter pylori assoziierter Gastritis oder zur Behandlung von Hauttumoren, Nebennierentumoren oder Lungentumoren.

## Revendications

1. Un polynucléotide codant pour une protéine membranaire TIRC7 d'approximativement 75kDa ou un fragment de celle-ci capable d'inhiber la prolifération des cellules T en réponse à l'alloactivation dans une culture de lymphocytes mixtes ou en réponse à des mitogènes, et comprenant une séquence d'ADN choisie dans le groupe consistant de :
(i) les séquences d'ADN comprenant une séquence nucléotide codant pour la séquence d'acide aminé représentée à la SEQ ID NO.2 ou à la SEQ ID NO.13 de la position d'acide aminé 1 à 614 ou de la position d'acide aminé 1 à 601;
(ii) les séquences d'ADN comprenant la séquence nucléotide représentée à la SEQ ID NO.1 ou à la SEQ ID NO.12;
(iii)les séquences d'ADN comprenant une séquence nucléotide codant pour un fragment de la protéine codée par les séquences d'ADN (i) ou (ii); dans lesquelles ledit fragment est capable d'inhiber la prolifération des cellules T en réponse à l'alloactivation dans une culture de lymphocytes mixtes ou en réponse à des mitogènes; et
(iv)les séquences d'ADN dont le brin complémentaire s'hybride dans des conditions d'hybridation stringentes avec le polynucléotide comme défini dans l'une quelconque de (i) à (iii) et qui sont au moins 70% identique au dit polynucléotide.

2. Utilisation d'une molécule d'acide nucléique d'au moins 15 nucléotides de long s'hybridant spécifiquement avec un polynucléotide de la revendication 1 ou avec un brin complémentaire de celui-ci pour la détection et/ou l'amplification d'un polynucléotide de la revendication 1 ou pour la répression de l'expression d'un gène comprenant le polynucléotide de la revendication 1, in vitro.

3. Un vecteur comprenant le polynucléotide de la revendication 1.

4. Le vecteur de la revendication 3, dans lequel ledit polynucléotide est lié de façon opérationnelle à des séquences régulatrices permettant la transcription et optionnellement l'expression de ladite molécule d'acide nucléique.

5. Une cellule hôte comprenant un polynucléotide de la revendication 1 sous le control d'un promoteur hétérologue ou le vecteur de la revendication 3 ou 4.

6. Une méthode pour la production d'une protéine membranaire TIRC7 ou d'un fragment biologiquement actif de celle-ci, comprenant:
(a) la cultivation de la cellule hôte de la revendication 5 dans des conditions permettant l'expression de la protéine; ou
(b) la traduction in vitro du polynucléotide de la revendication 1 ;
et la récupération de la protéine produite dans (a) ou (b).

7. Une protéine membranaire TIRC7 ou un fragment de celle-ci capable d'inhiber la prolifération des cellules T en réponse à l'alloactivation dans une culture de lymphocytes mixtes ou en réponse à des mitogènes, et codée par le polynucléotide de la revendication 1 ou produite par la méthode de la revendication 6.

8. Un anticorps reconnaissant spécifiquement un domaine extracellulaire de la protéine membranaire TIRC7 de la revendication 7.

9. Une cellule qui a été modifiée pour exprimer l'anticorps de la revendication 8.

10. Une composition pharmaceutique comprenant un peptide ou polypeptide qui est capable d'inhiber la stimulation des cellules T par l'intermédiaire de la protéine membranaire TIRC7 telle que définie dans la revendication 7 et/ou qui est reconnu par un anticorps capable d'inhiber la stimulation des cellules T par l'intermédiaire de la protéine membranaire TIRC7 codée par un fragment du polynucléotide de la revendication 1 ou un anticorps reconnaissant spécifiquement ledit peptide ou polypeptide, et optionnellement un véhicule acceptable sur le plan pharmaceutique.

11. La composition pharmaceutique de la revendication 10, dans laquelle le ledit peptide ou polypeptide est sous une forme soluble.

12. La composition pharmaceutique de la revendication 10 ou 11, dans laquelle le ledit peptide ou polypeptide comprend la séquence d'acide aminé représentée dans l'une quelconque des SEQ ID NO.3 à 9.

13. La composition pharmaceutique de l'une quelconque de revendications 10 à 12 comprenant en outre un deuxième agent qui inhibe la stimulation des cellules T.

14. La composition pharmaceutique de la revendication 13, dans laquelle le deuxième agent est un anticorps d'un récepteur ou d'un ligand impliqué dans la stimulation des cellules T ou est une forme soluble dudit récepteur.

15. La composition pharmaceutique de la revendication 14, dans laquelle ledit deuxième agent bloque l'interaction du récepteur de surface du CD2, du CD28, du CTLA4 ou du CD40 avec un ligand du CD2, du CD28, du CTLA4 ou du CD40.

16. La composition pharmaceutique de l'une quelconque de revendications 10 à 15 destinée à une utilisation dans la transplantation de cellules ou d'organes, pour le traitement des maladies auto-immunes, allergiques ou infectieuses, ou pour le traitement des tumeurs.

17. Une composition diagnostique comprenant un polynucléotide de la revendication 1, le vecteur de la revendication 3 ou 4, la cellule de la revendication 5 ou 9, la protéine de la revendication 7, ou l'anticorps de la revendication 8 ou un (poly)peptide ou anticorps comme défini dans l'une quelconque de revendications 10 à 12; et optionnellement des moyens adéquats pour la détection.

18. Une méthode in vitro pour induire ou maintenir une non-réponse d'une cellule T à un antigène comprenant la mise en contact de la cellule T avec un agent qui inhibe la stimulation de la cellule T par l'intermédiaire de la protéine membranaire TIRC7 de la revendication 7.

19. Utilisation d'un agent qui inhibe la stimulation des cellules T par l'intermédiaire de la protéine membranaire TIRC7 de la revendication 7 pour la préparation d'une composition pharmaceutique pour induire ou maintenir une non-réponse des cellules T à un antigène chez un sujet.

20. La méthode de revendication 18 ou l'utilisation de la revendication 19, dans laquelle l'agent bloque une interaction de la protéine membranaire TIRC7 avec son ligand.

21. La méthode ou l'utilisation de la revendication 20, dans laquelle l'agent est un peptide ou un polypeptide qui est capable d'inhiber la stimulation des cellules T par l'intermédiaire de la protéine membranaire TIRC7 et/ou qui est reconnu par un anticorps capable d'inhiber la stimulation de cellules T par l'intermédiaire de la protéine membranaire TIRC7 codée par un fragment du polynucléotide de la revendication 1 ou un anticorps reconnaissant spécifiquement ledit peptide ou polypeptide.

22. La méthode de l'une quelconque de revendications 18, 20 ou 22 ou l'utilisation de l'une quelconque de revendications 19 à 21, comprenant en outre l'utilisation d'un deuxième agent comme défini dans l'une quelconque de revendications 13 à 16.

23. Une composition pharmaceutique comprenant un premier agent qui stimule une cellule T par l'intermédiaire de la protéine membranaire TIRC7 de la revendication 7 et optionnellement un véhicule acceptable sur le plan pharmaceutique.

24. La composition pharmaceutique de la revendication 23, dans laquelle ledit agent est un ligand de la protéine membranaire TIRC7 ou est au moins un anticorps anti-protéine membranaire TIRC7.

25. La composition pharmaceutique de la revendication 23 à 24, comprenant en outre un deuxième agent qui stimule la prolifération de cellules T.

26. La composition pharmaceutique de la revendication 25, dans laquelle ledit agent est IL-2, IL-4 ou un agent qui stimule une cellule T par l'intermédiaire d'un récepteur de surface du CD2, du CD28, du CD40 ou du CTLA4.

27. Une méthode in vitro pour rétablir la réponse à un antigène d'une cellule T qui présente une non-réponse à l'antigène, comprenant la mise en contact de la cellule T, en présence de l'antigène, avec un premier agent qui stimule la cellule T par l'intermédiaire de la protéine membranaire TIRC7 de la revendication 7.

28. La méthode de la revendication 27, dans laquelle le ligand du TIRC7 est exprimé sur la surface des cellules, par l'introduction dans la cellule, d'une molécule d'acide nucléique codant pour le ligand du TIRC7, sous une forme convenant à l'expression du ligand du TIRC7 sur la surface des cellules.

29. La méthode de la revendication 28, dans laquelle la cellule est une cellule tumorale.

30. La méthode de l'une quelconque de revendications 27 à 29, comprenant en outre la mise en contact de la cellule T avec un deuxième agent comme défini dans la revendication 25 ou 26.

31. La méthode de la revendication 30, dans laquelle la cellule T est mise en contact avec le deuxième agent avant d'être mise en contact avec le premier agent.

32. La méthode de la revendication 30 ou 31, dans laquelle un ligand du CD2, du CD28, du CD40 ou du CTLA4 est exprimé sur la surface des cellules, par l'introduction dans la cellule, d'une molécule d'acide nucléique codant pour le ligand du CD2, du CD28, du CD40 ou du CTLA4, sous une forme convenant à l'expression dudit ligand sur la surface des cellules.

33. Utilisation d'un premier agent comme défini à la revendication 23 à 24 pour la préparation d'une composition pharmaceutique destinée à stimuler une réponse des cellules T à une cellule tumorale chez un sujet présentant une tumeur.

34. L'utilisation de la revendication 33, pour laquelle la cellule tumorale est modifiée pour exprimer un ligand du TIRC7 et/ou un ligand du CD2, du CD28, du CD40 ou du CTLA4, de préférence sur la surface des cellules tumorales.

35. L'utilisation de la revendication 33 ou 34, pour laquelle la cellule tumorale est une cellule tumorale modifiée provenant du sujet.

36. L'utilisation de l'une quelconque de revendications 19 à 22 ou 33 à 35, pour laquelle le sujet est un receveur de greffons de cellules de souches périphériques ou de moelle osseuse.

37. L'utilisation de la revendication 36, pour laquelle la composition pharmaceutique est conçue pour être mise en contact avec les cellules de souches périphériques ou les cellules de moelle osseuse avant transplantation chez le receveur.

38. Une méthode pour l'identification de composés qui activent ou qui co-stimulent les cellules T ou pour l'identification d'inhibiteurs de l'activation et de la stimulation des cellules T comprenant
(a) la cultivation des cellules T en présence de la protéine de la revendication de 7, de l'anticorps de la revendication 8, de la cellule de la revendication 5 ou 9 ou d'un peptide ou polypeptide qui est capable d'inhiber la stimulation des cellules T par l'intermédiaire de la protéine membranaire TIRC7 et/ou qui est reconnu par un anticorps capable d'inhiber la stimulation des cellules T par l'intermédiaire de la protéine membranaire TIRC7 codée par un fragment du polynucléotide de la revendication 1 ou un anticorps reconnaissant spécifiquement ledit peptide ou polypeptide et, optionnellement, en présence d'un composant capable de fournir un signal détectable en réponse à l'activation des cellules T, avec un composé à tester dans des conditions permettant l'interaction du composé avec le (poly)peptide, l'anticorps ou le(s) cellule(s); et
(b) la détection de la présence ou de l'absence d'un signal produit par l'interaction du composé avec les cellules.

39. Utilisation d'un peptide ou polypeptide qui est capable d'inhiber la stimulation des cellules T par l'intermédiaire de la protéine membranaire TIRC7 et/ou qui est reconnu par un anticorps capable d'inhiber la stimulation des cellules T par l'intermédiaire de la protéine membranaire TIRC7 codée par un fragment du polynucléotide de la revendication 1 ou un anticorps reconnaissant spécifiquement ledit peptide ou polypeptide, le polynucléotide de la revendication 1, le vecteur de la revendication 3 ou 4, la protéine de la revendication 7, l'anticorps de la revendication 8, la cellule de la revendication 5 ou 9 ou le composé identifié selon la méthode de la revendication 38 pour la préparation d'une composition pharmaceutique destinée au traitement d'affections aiguës et chroniques, impliquant l'activation des cellules T et des réponses immunitaires Th1 et Th2, pour le traitement du rejet aigu et chronique d'allo- et xeno-greffons d'organes et dans la transplantation de moelle osseuse, pour le traitement de la polyarthrite rhumatoïde, du lupus érythémateux, de la sclérose en plaques, de l'encéphalite, de la vasculite, du diabète sucré, de la pancréatite, de la gastrite, de la thyroïdite, pour le traitement des affections malignes des cellules T, B ou NK, pour le traitement de l'asthme, de la lepromatose, de la gastrite associée au Helicobacter pylori ou pour le traitement des tumeurs de la peau, des tumeurs des surrénales ou des tumeurs du poumon.


### SEQUENCE LISTING

(1) GENERAL INFORMATION:
   (i) APPLICANT:
      (A) NAME: Dr. Utku
      (B) STREET: Knesebeckstr. 5
      (C) CITY: Berlin
      (D) STATE: none
      (E) COUNTRY: Germany
      (F) POSTAL CODE (ZIP): 10623
   (ii) TITLE OF INVENTION: A novel T-cell membrane protein (TIRC7) and uses thereof
   (iii) NUMBER OF SEQUENCES: 13
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30 (EPO)
(2) INFORMATION FOR SEQ ID NO: 1:
   (i) SEQUENCE CHARACTERISTICS:
      (A) LENGTH: 2488 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (ix) FEATURE:
      (A) NAME/KEY: CDS
      (B) LOCATION:537..2378
   (xi) SEQUENCE DESCRIPTION: SEQ ID NO: 1:
   GGACGCCCCT GCTCCAGGCC CCCGGGGGGC CGCACCAGGA CCTGAGGGTC AAGTGAGTGA     60
   GGGATGACCT CATGCCCTTT CTGGCCAGCC CAGAACCCCT GGCCAGTCGC TGGGCTGGGC    120
   CAGGCTGAGC TCCGACTCCT TGTCCAGTGC TCTCCCCAGG CTGGCCCCGC CTCCTCCTTC    180
   AGGCCCGGAA CTTCCCACAG TCCCAAGCCC TAGCCCTAGG GGGTTCTCCT CTTCTGGTCC    240
   TGCCCGGGAG GCCTCCTGCC TTCCCCTGTG GGCAGGGCCA GTGTGCCCAA TTGCCCGATT    300
   GCCCGTGCTG GGCAGGGTCC TGCCCGGGGG GCCTGGTGGG GGAGGCAGGG CAGGAGGTTG    360
   GAGCAGCCCT GCCCAGCCCC GTGGCCGCCA GCTTTGTGGC AGGTGCCGTG GAGCCCCACA      420
   AGGCCCCTGC CCTAGAGCGC CTGCTCTGGA GGGCCTGCCG CGGCTTCCTC ATTGCCAGCT      480
   TCAGGGAGCT GGAGCAGCCG CTGGAGCACC CCGTGACGGG CGAGCCAGCC ACGTGG          536
   ATG ACC TTC CTC ATC TCC TAC TGG GGT GAG CAG ATC GGA CAG AAG ATC        584
   Met Thr Phe Leu Ile Ser Tyr Trp Gly Glu Gln Ile Gly Gln Lys Ile
     1               5                  10                  15
   CGC AAG ATC ACG GAC TGC TTC CAC TGC CAC GTC TTC CCG TTT CTG CAG        632
   Arg Lys Ile Thr Asp Cys Phe His Cys His Val Phe Pro Phe Leu Gln
                    20                  25                  30
   CAG GAG GAG GCC CGC CTC GGG GCC CTG CAG CAG CTG CAA CAG CAG AGC        680
   Gln Glu Glu Ala Arg Leu Gly Ala Leu Gln Gln Leu Gln Gln Gln Ser
                35                  40                  45
   CAG GAG CTG CAG GAG GTC CTC GGG GAG ACA GAG CGG TTC CTG AGC CAG        728
   Gln Glu Leu Gln Glu Val Leu Gly Glu Thr Glu Arg Phe Leu Ser Gln
            50                  55                  60
   GTG CTA GGC CGG GTG CTG CAG CTG CTG CCG CCA GGG CAG GTG CAG GTC        776
   Val Leu Gly Arg Val Leu Gln Leu Leu Pro Pro Gly Gln Val Gln Val
    65                  70                  75                  80
   CAC AAG ATG AAG GCC GTG TAC CTG GCC CTG AAC CAG TGC AGC GTG AGC        824
   His Lys Met Lys Ala Val Tyr Leu Ala Leu Asn Gln Cys Ser Val Ser
                    85                  90                  95
   ACC ACG CAC AAG TGC CTC ATT GCC GAG GCC TGG TGC TCT GTG CGA GAC        872
   Thr Thr His Lys Cys Leu Ile Ala Glu Ala Trp Cys Ser Val Arg Asp
               100                 105                 110
   CTG CCC GCC CTG CAG GAG GCC CTG CGG GAC AGC TCG ATG GAG GAG GGA        920
   Leu Pro Ala Leu Gln Glu Ala Leu Arg Asp Ser Ser Met Glu Glu Gly
           115                 120                 125
   GTG AGT GCC GTG GCT CAC CGC ATC CCC TGC CGG GAC ATG CCC CCC ACA        968
   Val Ser Ala Val Ala His Arg Ile Pro Cys Arg Asp Met Pro Pro Thr
       130                 135                 140
   CTC ATC CGC ACC AAC CGC TTC ACG GCC AGC TTC CAG GGC ATC GTG GAT       1016
   Leu Ile Arg Thr Asn Arg Phe Thr Ala Ser Phe Gln Gly Ile Val Asp
   145                 150                 155                 160
   GCC TAC GGC GTG GGC CGC TAC CAG GAG GTC AAC CCC GCT CCC TAC ACC       1064
   Ala Tyr Gly Val Gly Arg Tyr Gln Glu Val Asn Pro Ala Pro Tyr Thr
                   165                 170                 175
   ATC ATC ACC TTC CCC TTC CTG TTT GCT GTG ATG TTC GGG GAT GTG GGC       1112
   Ile Ile Thr Phe Pro Phe Leu Phe Ala Val Met Phe Gly Asp Val Gly
               180                 185                 190
   CAC GGG CTG CTC ATG TTC CTC TTC GCC CTG GCC ATG GTC CTT GCG GAG       1160
   His Gly Leu Leu Met Phe Leu Phe Ala Leu Ala Met Val Leu Ala Glu
           195                    200                    205
   AAC CGA CCG GCT GTG AAG GCC GCG CAG AAC GAG ATC TGG CAG ACT TTC      1208
   Asn Arg Pro Ala Val Lys Ala Ala Gln Asn Glu Ile Trp Gln Thr Phe
       210                    215                    220
   TTC AGG GGC CGC TAC CTG CTC CTG CTT ATG GGC CTG TTC TCC ATC TAC      1256
   Phe Arg Gly Arg Tyr Leu Leu Leu Leu Met Gly Leu Phe Ser Ile Tyr
   225                    230                    235                    240
   ACC GGC TTC ATC TAC AAC GAG TGC TTC AGT CGC GCC ACC AGC ATC TTC      1304
   Thr Gly Phe Ile Tyr Asn Glu Cys Phe Ser Arg Ala Thr Ser Ile Phe
                   245                    250                    255
   CCC TCG GGC TGG AGT GTG GCC GCC ATG GCC AAC CAG TCT GGC TGG AGT      1352
   Pro Ser Gly Trp Ser Val Ala Ala Met Ala Asn Gln Ser Gly Trp Ser
               260                    265                    270
   GAT GCA TTC CTG GCC CAG CAC ACG ATG CTT ACC CTG GAT CCC AAC GTC      1400
   Asp Ala Phe Leu Ala Gln His Thr Met Leu Thr Leu Asp Pro Asn Val
           275                    280                    285
   ACC GGT GTC TTC CTG GGA CCC TAC CCC TTT GGC ATC GAT CCT ATT TGG      1448
   Thr Gly Val Phe Leu Gly Pro Tyr Pro Phe Gly Ile Asp Pro Ile Trp
       290                    295                    300
   AGC CTG GCT GCC AAC CAC TTG AGC TTC CTC AAC TCC TTC AAG ATG AAG      1496
   Ser Leu Ala Ala Asn His Leu Ser Phe Leu Asn Ser Phe Lys Met Lys
   305                    310                    315                    320
   ATG TCC GTC ATC CTG GGC GTC GTG CAC ATG GCC TTT GGG GTG GTC CTC      1544
   Met Ser Val Ile Leu Gly Val Val His Met Ala Phe Gly Val Val Leu
                   325                    330                    335
   GGA GTC TTC AAC CAC GTG CAC TTT GGC CAG AGG CAC CGG CTG CTG CTG      1592
   Gly Val Phe Asn His Val His Phe Gly Gln Arg His Arg Leu Leu Leu
               340                    345                    350
   GAG ACG CTG CCG GAG CTC ACC TTC CTG CTG GGA CTC TTC GGT TAC CTC      1640
   Glu Thr Leu Pro Glu Leu Thr Phe Leu Leu Gly Leu Phe Gly Tyr Leu
           355                    360                    365
   GTG TTC CTA GTC ATC TAC AAG TGG CTG TGT GTC TGG GCT GCC AGG GCC      1688
   Val Phe Leu Val Ile Tyr Lys Trp Leu Cys Val Trp Ala Ala Arg Ala
       370                    375                    380
   GCC TCG GCC CCC AGC ATC CTC ATC CAC TTC ATC AAC ATG TTC CTC TTC      1736
   Ala Ser Ala Pro Ser Ile Leu Ile His Phe Ile Asn Met Phe Leu Phe
   385                    390                    395                    400
   TCC CAC AGC CCC AGC AAC AGG CTG CTC TAC CCC CGG CAG GAG GTG GTC      1784
   Ser His Ser Pro Ser Asn Arg Leu Leu Tyr Pro Arg Gln Glu Val Val
                   405                    410                    415
   CAG GCC ACG CTG GTG GTC CTG GCC TTG GCC ATG GTG CCC ATC CTG CTG        1832
   Gln Ala Thr Leu Val Val Leu Ala Leu Ala Met Val Pro Ile Leu Leu
               420                 425                 430
   CTT GGC ACA CCC CTG CAC CTG CTG CAC CGC CAC CGC CGC CGC CTG CGG        1880
   Leu Gly Thr Pro Leu His Leu Leu His Arg His Arg Arg Arg Leu Arg
           435                 440                 445
   AGG AGG CCC GCT GAC CGA CAG GAG GAA AAC AAG GCC GGG TTG CTG GAC        1928
   Arg Arg Pro Ala Asp Arg Gln Glu Glu Asn Lys Ala Gly Leu Leu Asp
       450                 455                 460
   CTG CCT GAC GCA TCT GTG AAT GGC TGG AGC TCC GAT GAG GAA AAG GCA        1976
   Leu Pro Asp Ala Ser Val Asn Gly Trp Ser Ser Asp Glu Glu Lys Ala
   465                 470                 475                 480
   GGG GGC CTG GAT GAT GAA GAG GAG GCC GAG CTC GTC CCC TCC GAG GTG        2024
   Gly Gly Leu Asp Asp Glu Glu Glu Ala Glu Leu Val Pro Ser Glu Val
                   485                 490                 495
   CTC ATG CAC CAG GCC ATC CAC ACC ATC GAG TTC TGC CTG GGC TGC GTC        2072
   Leu Met His Gln Ala Ile His Thr Ile Glu Phe Cys Leu Gly Cys Val
               500                 505                 510
   TCC AAC ACC GCC TCC TAC CTG CGC CTG TGG GCC CTG AGC CTG GCC CAC        2120
   Ser Asn Thr Ala Ser Tyr Leu Arg Leu Trp Ala Leu Ser Leu Ala His
           515                 520                 525
   GCC CAG CTG TCC GAG GTT CTG TGG GCC ATG GTG ATG CGC ATA GGC CTG        2168
   Ala Gln Leu Ser Glu Val Leu Trp Ala Met Val Met Arg Ile Gly Leu
       530                 535                 540
   GGC CTG GGC CGG GAG GTG GGC GTG GCG GCT GTG GTG CTG GTC CCC ATC        2216
   Gly Leu Gly Arg Glu Val Gly Val Ala Ala Val Val Leu Val Pro Ile
   545                 550                 555                 560
   TTT GCC GCC TTT GCC GTG ATG ACC GTG GCT ATC CTG CTG GTG ATG GAG        2264
   Phe Ala Ala Phe Ala Val Met Thr Val Ala Ile Leu Leu Val Met Glu
                   565                 570                 575
   GGA CTC TCA GCC TTC CTG CAC GCC CTG CGG CTG CAC TGG GTG GAA TTC        2312
   Gly Leu Ser Ala Phe Leu His Ala Leu Arg Leu His Trp Val Glu Phe
               580                 585                 590
   CAG AAC AAG TTC TAC TCA GGC ACG GGC TAC AAG CTG AGT CCC TTC ACC        2360
   Gln Asn Lys Phe Tyr Ser Gly Thr Gly Tyr Lys Leu Ser Pro Phe Thr
           595                 600                 605
   TTC GCT GCC ACA GAT GAC TAGGGCCCAC TGCAGGTCCT GCCAGACCTC               2408
   Phe Ala Ala Thr Asp Asp
       610
   CTTCCTGACC TCTGAGGCAG GAGAGGAATA AAGACGGTCC GCCCTGGCAA AAAAAAAAAA      2468
   AAAAAAAAAA AAAAAAAAAA                                                  2488
(2) INFORMATION FOR SEQ ID NO: 2:
   (i) SEQUENCE CHARACTERISTICS:
      (A) LENGTH: 614 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Thr Phe Leu Ile Ser Tyr Trp Gly Glu Gln Ile Gly Gln Lys Ile
   1 5 10 15
   Arg Lys Ile Thr Asp Cys Phe His Cys His Val Phe Pro Phe Leu Gln
   20 25 30
   Gln Glu Glu Ala Arg Leu Gly Ala Leu Gln Gln Leu Gln Gln Gln Ser
   35 40 45
   Gln Glu Leu Gln Glu Val Leu Gly Glu Thr Glu Arg Phe Leu Ser Gln
   50 55 60
   Val Leu Gly Arg Val Leu Gln Leu Leu Pro Pro Gly Gln Val Gln Val
   65 70 75 80
   His Lys Met Lys Ala Val Tyr Leu Ala Leu Asn Gln Cys Ser Val Ser
   85 90 95
   Thr Thr His Lys Cys Leu Ile Ala Glu Ala Trp Cys Ser Val Arg Asp
   100 105 110
   Leu Pro Ala Leu Gln Glu Ala Leu Arg Asp Ser Ser Met Glu Glu Gly
   115 120 125
   Val Ser Ala Val Ala His Arg Ile Pro Cys Arg Asp Met Pro Pro Thr
   130 135 140
   Leu Ile Arg Thr Asn Arg Phe Thr Ala Ser Phe Gln Gly Ile Val Asp
   145 150 155 160
   Ala Tyr Gly Val Gly Arg Tyr Gln Glu Val Asn Pro Ala Pro Tyr Thr
   165 170 175
   Ile Ile Thr Phe Pro Phe Leu Phe Ala Val Met Phe Gly Asp Val Gly
   180 185 190
   His Gly Leu Leu Met Phe Leu Phe Ala Leu Ala Met Val Leu Ala Glu
   195 200 205
   Asn Arg Pro Ala Val Lys Ala Ala Gln Asn Glu Ile Trp Gln Thr Phe
   210 215 220
   Phe Arg Gly Arg Tyr Leu Leu Leu Leu Met Gly Leu Phe Ser Ile Tyr
   225 230 235 240
   Thr Gly Phe Ile Tyr Asn Glu Cys Phe Ser Arg Ala Thr Ser Ile Phe
   245 250 255
   Pro Ser Gly Trp Ser Val Ala Ala Met Ala Asn Gln Ser Gly Trp Ser
   260 265 270
   Asp Ala Phe Leu Ala Gln His Thr Met Leu Thr Leu Asp Pro Asn Val
   275 280 285
   Thr Gly Val Phe Leu Gly Pro Tyr Pro Phe Gly Ile Asp Pro Ile Trp
   290 295 300
   Ser Leu Ala Ala Asn His Leu Ser Phe Leu Asn Ser Phe Lys Met Lys
   305 310 315 320
   Met Ser Val Ile Leu Gly Val Val His Met Ala Phe Gly Val Val Leu
   325 330 335
   Gly Val Phe Asn His Val His Phe Gly Gln Arg His Arg Leu Leu Leu
   340 345 350
   Glu Thr Leu Pro Glu Leu Thr Phe Leu Leu Gly Leu Phe Gly Tyr Leu
   355 360 365
   Val Phe Leu Val Ile Tyr Lys Trp Leu Cys Val Trp Ala Ala Arg Ala
   370 375 380
   Ala Ser Ala Pro Ser Ile Leu Ile His Phe Ile Asn Met Phe Leu Phe
   385 390 395 400
   Ser His Ser Pro Ser Asn Arg Leu Leu Tyr Pro Arg Gln Glu Val Val
   405 410 415
   Gln Ala Thr Leu Val Val Leu Ala Leu Ala Met Val Pro Ile Leu Leu
   420 425 430
   Leu Gly Thr Pro Leu His Leu Leu His Arg His Arg Arg Arg Leu Arg
   435 440 445
   Arg Arg Pro Ala Asp Arg Gln Glu Glu Asn Lys Ala Gly Leu Leu Asp
   450 455 460
   Leu Pro Asp Ala Ser Val Asn Gly Trp Ser Ser Asp Glu Glu Lys Ala
   465 470 475 480
   Gly Gly Leu Asp Asp Glu Glu Glu Ala Glu Leu Val Pro Ser Glu Val
   485 490 495
   Leu Met His Gln Ala Ile His Thr Ile Glu Phe Cys Leu Gly Cys Val
   500 505 510
   Ser Asn Thr Ala Ser Tyr Leu Arg Leu Trp Ala Leu Ser Leu Ala His
   515 520 525
   Ala Gln Leu Ser Glu Val Leu Trp Ala Met Val Met Arg Ile Gly Leu
       530                 535                 540
   Gly Leu Gly Arg Glu Val Gly Val Ala Ala Val Val Leu Val Pro Ile
   545                 550                 555                 560
   Phe Ala Ala Phe Ala Val Met Thr Val Ala Ile Leu Leu Val Met Glu
                   565                 570                 575
   Gly Leu Ser Ala Phe Leu His Ala Leu Arg Leu His Trp Val Glu Phe
               580                 585                 590
   Gln Asn Lys Phe Tyr Ser Gly Thr Gly Tyr Lys Leu Ser Pro Phe Thr
           595                 600                 605
   Phe Ala Ala Thr Asp Asp
       610
(2) INFORMATION FOR SEQ ID NO: 3:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 3 :
   Arg Arg Pro Ala Asp Arg Gln Glu Glu Asn Lys Ala Gly
   1               5                   10
(2) INFORMATION FOR SEQ ID NO: 4:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 4:
   Ser Ser Asp Glu Glu Lys Ala Gly Gly Leu Asp Asp Glu Glu
   1 5 10
(2) INFORMATION FOR SEQ ID NO: 5:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 5:
   Val Glu Phe Gln Asn Lys Phe Tyr Ser Gly Thr Gly Tyr Lys
   1 5 10
(2) INFORMATION FOR SEQ ID NO: 6:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 6 :
   Ser Gly Thr Gly Tyr Lys Leu Ser Pro Phe Thr Phe Ala Ala Thr Asp
   1 5 10 15
(2) INFORMATION FOR SEQ ID NO: 7 :
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 7:
   Asp Leu Pro Asp Ala Ser Val Asn Gly Trp Ser Ser Asp Glu
   1               5                   10
(2) INFORMATION FOR SEQ ID NO: 8:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 8:
(2) INFORMATION FOR SEQ ID NO: 9:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 9:
   Ala Asn Gln Ser Gly Trp Ser Asp Ala Phe Leu Ala Gln His
   1               5                   10
(2) INFORMATION FOR SEQ ID NO: 10:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (iii) HYPOTHETICAL: YES
   (xi) SEQUENCE DESCRIPTION: SEQ ID NO: 10:
   GACGGAACAG CTTC
(2) INFORMATION FOR SEQ ID NO: 11:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (iii) HYPOTHETICAL: YES
   (xi) SEQUENCE DESCRIPTION: SEQ ID NO: 11:
   TGCGTCTGGT TCT                                                    13
(2) INFORMATION FOR SEQ ID NO : 12 :
   (i) SEQUENCE CHARACTERISTICS:
      (A) LENGTH: 2488 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (ix) FEATURE:
      (A) NAME/KEY: CDS
      (B) LOCATION:537..2378
   (xi) SEQUENCE DESCRIPTION: SEQ ID NO: 12:
   GGACGCCCCT GCTCCAGGCC CCCGGGGGGC CGCACCAGGA CCTGAGGGTC AAGTGAGTGA    60
   GGGATGACCT CATGCCCTTT CTGGCCAGCC CAGAACCCCT GGCCAGTCGC TGGGCTGGGC   120
   CAGGCTGAGC TCCGACTCCT TGTCCAGTGC TCTCCCCAGG CTGGCCCCGC CTCCTCCTTC   180
   AGGCCCGGAA CTTCCCACAG TCCCAAGCCC TAGCCCTAGG GGGTTCTCCT CTTCTGGTCC   240
   TGCCCGGGAG GCCTCCTGCC TTCCCCTGTG GGCAGGGCCA GTGTGCCCAA TTGCCCGATT   300
   GCCCGTGCTG GGCAGGGTCC TGCCCGGGGG GCCTGGTGGG GGAGGCAGGG CAGGAGGTTG   360
   GAGCAGCCCT GCCCAGCCCC GTGGCCGCCA GCTTTGTGGC AGGTGCCGTG GAGCCCCACA   420
   AGGCCCCTGC CCTAGAGCGC CTGCTCTGGA GGGCCTGCCG CGGCTTCCTC ATTGCCAGCT   480
   TCAGGGAGCT GGAGCAGCCG CTGGAGCACC CCGTGACGGG CGAGCCAGCC ACGTGG       536
   ATG ACC TTC CTC ATC TCC TAC TGG GGT GAG CAG ATC GGA CAG AAG ATC     584
   Met Thr Phe Leu Ile Ser Tyr Trp Gly Glu Gln Ile Gly Gln Lys Ile
     1               5                  10                  15
   CGC AAG ATC ACG GAC TGC TTC CAC TGC CAC GTC TTC CCG TTT CTG CAG     632
   Arg Lys Ile Thr Asp Cys Phe His Cys His Val Phe Pro Phe Leu Gln
                    20                  25                  30
   CAG GAG GAG GCC CGC CTC GGG GCC CTG CAG CAG CTG CAA CAG CAG AGC     680
   Gln Glu Glu Ala Arg Leu Gly Ala Leu Gln Gln Leu Gln Gln Gln Ser
                35                  40                  45
   CAG GAG CTG CAG GAG GTC CTC GGG GAG ACA GAG CGG TTC CTG AGC CAG     728
   Gln Glu Leu Gln Glu Val Leu Gly Glu Thr Glu Arg Phe Leu Ser Gln
        50                  55                  60
   GTG CTA GGC CGG GTG CTG CAG CTG CTG CCG CCA GGG CAG GTG CAG GTC     776
   Val Leu Gly Arg Val Leu Gln Leu Leu Pro Pro Gly Gln Val Gln Val
    65                  70                  75                  80
   CAC AAG ATG AAG GCC GTG TAC CTG GCC CTG AAC CAG TGC AGC GTG AGC     824
   His Lys Met Lys Ala Val Tyr Leu Ala Leu Asn Gln Cys Ser Val Ser
                    85                  90                  95
   ACC ACG CAC AAG TGC CTC ATT GCC GAG GCC TGG TGC TCT GTG CGA GAC     872
   Thr Thr His Lys Cys Leu Ile Ala Glu Ala Trp Cys Ser Val Arg Asp
               100                 105                 110
   CTG CCC GCC CTG CAG GAG GCC CTG CAG GAC AGC TCG ATG GAG GAG GGA     920
   Leu Pro Ala Leu Gln Glu Ala Leu Gln Asp Ser Ser Met Glu Glu Gly
           115                 120                 125
   GTG AGT GCC GTG GCT CAC CGC ATC CCC TGC CGG GAC ATG CCC CCC ACA     968
   Val Ser Ala Val Ala His Arg Ile Pro Cys Arg Asp Met Pro Pro Thr
       130                 135                 140
   CTC ATC CGC ACC AAC CGC TTC ACG GCC AGC TTC CAG GGC ATC GTG GAT    1016
   Leu Ile Arg Thr Asn Arg Phe Thr Ala Ser Phe Gln Gly Ile Val Asp
   145                 150                 155                 160
   GCC TAC GGC GTG GGC CGC TAC CAG GAG GTC AAC CCC GCT CCC TAC ACC    1064
   Ala Tyr Gly Val Gly Arg Tyr Gln Glu Val Asn Pro Ala Pro Tyr Thr
                   165                 170                 175
   ATC ATC ACC TTC CCC TTC CTG TTT GCT GTG ATG TTC GGG GAT GTG GGC    1112
   Ile Ile Thr Phe Pro Phe Leu Phe Ala Val Met Phe Gly Asp Val Gly
               180                 185                 190
   CAC GGG CTG CTC ATG TTC CTC TTC GCC CTG GCC ATG GTC CTT GCG GAG    1160
   His Gly Leu Leu Met Phe Leu Phe Ala Leu Ala Met Val Leu Ala Glu
           195                 200                 205
   AAC CGA CCG GCT GTG AAG GCC GCG CAG AAC GAG ATC TGG CAG ACT TTC    1208
   Asn Arg Pro Ala Val Lys Ala Ala Gln Asn Glu Ile Trp Gln Thr Phe
       210                 215                 220
   TTC AGG GGC CGC TAC CTG CTC CTG CTT ATG GGC CTG TTC TCC ATC TAC      1256
   Phe Arg Gly Arg Tyr Leu Leu Leu Leu Met Gly Leu Phe Ser Ile Tyr
   225                 230                 235                 240
   ACC GGC TTC ATC TAC AAC GAG TGC TTC AGT CGC GCC ACC AGC ATC TTC      1304
   Thr Gly Phe Ile Tyr Asn Glu Cys Phe Ser Arg Ala Thr Ser Ile Phe
                   245                 250                 255
   CCC TCG GGC TGG AGT GTG GCC GCC ATG GCC AAC CAG TCT GGC TGG AGT      1352
   Pro Ser Gly Trp Ser Val Ala Ala Met Ala Asn Gln Ser Gly Trp Ser
               260                 265                 270
   GAT GCA TTC CTG GCC CAG CAC ACG ATG CTT ACC CTG GAT CCC AAC GTC      1400
   Asp Ala Phe Leu Ala Gln His Thr Met Leu Thr Leu Asp Pro Asn Val
           275                 280                 285
   ACC GGT GTC TTC CTG GGA CCC TAC CCC TTT GGC ATC GAT CCT ATT TGG      1448
   Thr Gly Val Phe Leu Gly Pro Tyr Pro Phe Gly Ile Asp Pro Ile Trp
       290                 295                 300
   AGC CTG GCT GCC AAC CAC TTG AGC TTC CTC AAC TCC TTC AAG ATG AAG      1496
   Ser Leu Ala Ala Asn His Leu Ser Phe Leu Asn Ser Phe Lys Met Lys
   305                 310                 315                 320
   ATG TCC GTC ATC CTG GGC GTC GTG CAC ATG GCC TTT GGG GTG GTC CTC      1544
   Met Ser Val Ile Leu Gly Val Val His Met Ala Phe Gly Val Val Leu
                   325                 330                 335
   GGA GTC TTC AAC CAC GTG CAC TTT GGC CAG AGG CAC CGG CTG CTG CTG      1592
   Gly Val Phe Asn His Val His Phe Gly Gln Arg His Arg Leu Leu Leu
               340                 345                 350
   GAG ACG CTG CCG GAG CTC ACC TTC CTG CTG GGA CTC TTC GGT TAC CTC      1640
   Glu Thr Leu Pro Glu Leu Thr Phe Leu Leu Gly Leu Phe Gly Tyr Leu
           355                 360                 365
   GTG TTC CTA GTC ATC TAC AAG TGG CTG TGT GTC TGG GCT GCC AGG GCC      1688
   Val Phe Leu Val Ile Tyr Lys Trp Leu Cys Val Trp Ala Ala Arg Ala
       370                 375                 380
   GCC TCG GCC CCC AGC ATC CTC ATC CAC TTC ATC AAC ATG TTC CTC TTC      1736
   Ala Ser Ala Pro Ser Ile Leu Ile His Phe Ile Asn Met Phe Leu Phe
   385                 390                 395                 400
   TCC CAC AGC CCC AGC AAC AGG CTG CTC TAC CCC CGG CAG GAG GTG GTC      1784
   Ser His Ser Pro Ser Asn Arg Leu Leu Tyr Pro Arg Gln Glu Val Val
                   405                 410                 415
   CAG GCC ACG CTG GTG GTC CTG GCC TTG GCC ATG GTG CCC ATC CTG CTG      1832
   Gln Ala Thr Leu Val Val Leu Ala Leu Ala Met Val Pro Ile Leu Leu
               420                 425                 430
   CTT GGC ACA CCC CTG CAC CTG CTG CAC CGC CAC CGC CGC CGC CTG CGG      1880
   Leu Gly Thr Pro Leu His Leu Leu His Arg His Arg Arg Arg Leu Arg
   435 440 445
   AGG AGG CCC GCT GAC CGA CAG GAG GAA AAC AAG GCC GGG TTG CTG GAC 1928
   Arg Arg Pro Ala Asp Arg Gln Glu Glu Asn Lys Ala Gly Leu Leu Asp
   450 455 460
   CTG CCT GAC GCA TCT GTG AAT GGC TGG AGC TCC GAT GAG GAA AAG GCA 1976
   Leu Pro Asp Ala Ser Val Asn Gly Trp Ser Ser Asp Glu Glu Lys Ala
   465 470 475 480
   GGG GGC CTG GAT GAT GAA GAG GAG GCC GAG CTC GTC CCC TCC GAG GTG 2024
   Gly Gly Leu Asp Asp Glu Glu Glu Ala Glu Leu Val Pro Ser Glu Val
   485 490 495
   CTC ATG CAC CAG GCC ATC CAC ACC ATC GAG TTC TGC CTG GGC TGC GTC 2072
   Leu Met His Gln Ala Ile His Thr Ile Glu Phe Cys Leu Gly Cys Val
   500 505 510
   TCC AAC ACC GCC TCC TAC CTG CGC CTG TGG GCC CTG AGC CTG GCC CAC 2120
   Ser Asn Thr Ala Ser Tyr Leu Arg Leu Trp Ala Leu Ser Leu Ala His
   515 520 525
   GCC CAG CTG TCC GAG GTT CTG TGG GCC ATG GTG ATG CGC ATA GGC CTG 2168
   Ala Gln Leu Ser Glu Val Leu Trp Ala Met Val Met Arg Ile Gly Leu
   530 535 540
   GGC CTG GGC CGG GAG GTG GGC GTG GCG GCT GTG GTG CTG GTC CCC ATC 2216
   Gly Leu Gly Arg Glu Val Gly Val Ala Ala Val Val Leu Val Pro Ile
   545 550 555 560
   TTT GCC GCC TTT GCC GTG ATG ACC GTG GCT ATC CTG CTG GTG ATG GAG 2264
   Phe Ala Ala Phe Ala Val Met Thr Val Ala Ile Leu Leu Val Met Glu
   565 570 575
   GGA CTC TCA GCC TTC CTG CAC GCC CTG CGG CTG CAC TGG GTG GAA TTC 2312
   Gly Leu Ser Ala Phe Leu His Ala Leu Arg Leu His Trp Val Glu Phe
   580 585 590
   CAG AAC AAG TTC TAC TCA GGC ACG GGC TAC AAG CTG AGT CCC TTC ACC 2360
   Gln Asn Lys Phe Tyr Ser Gly Thr Gly Tyr Lys Leu Ser Pro Phe Thr
   595 600 605
   TTC GCT GCC ACA GAT GAC TAGGGCCCAC TGCAGGTCCT GCCAGACCTC 2408
   Phe Ala Ala Thr Asp Asp
   610
   CTTCCTGACC TCTGAGGCAG GAGAGGAATA AAGACGGTCC GCCCTGGCAA AAAAAAAAAA 2468
   AAAAAAAAAA AAAAAAAAAA 2488
(2) INFORMATION FOR SEQ ID NO: 13:
   (i) SEQUENCE CHARACTERISTICS:
      (A) LENGTH: 614 amino acids
      (B) TYPE: amino acid
      (D) TOPOLOGY: linear
   (ii) MOLECULE TYPE: protein
   (xi) SEQUENCE DESCRIPTION: SEQ ID NO: 13:
   Met Thr Phe Leu Ile Ser Tyr Trp Gly Glu Gln Ile Gly Gln Lys Ile
   1 5 10 15
   Arg Lys Ile Thr Asp Cys Phe His Cys His Val Phe Pro Phe Leu Gln
   20 25 30
   Gln Glu Glu Ala Arg Leu Gly Ala Leu Gln Gln Leu Gln Gln Gln Ser
   35 40 45
   Gln Glu Leu Gln Glu Val Leu Gly Glu Thr Glu Arg Phe Leu Ser Gln
   50 55 60
   Val Leu Gly Arg Val Leu Gln Leu Leu Pro Pro Gly Gln Val Gln Val
   65 70 75 80
   His Lys Met Lys Ala Val Tyr Leu Ala Leu Asn Gln Cys Ser Val Ser
   85 90 95
   Thr Thr His Lys Cys Leu Ile Ala Glu Ala Trp Cys Ser Val Arg Asp
   100 105 110
   Leu Pro Ala Leu Gln Glu Ala Leu Gln Asp Ser Ser Met Glu Glu Gly
   115 120 125
   Val Ser Ala Val Ala His Arg Ile Pro Cys Arg Asp Met Pro Pro Thr
   130 135 140
   Leu Ile Arg Thr Asn Arg Phe Thr Ala Ser Phe Gln Gly Ile Val Asp
   145 150 155 160
   Ala Tyr Gly Val Gly Arg Tyr Gln Glu Val Asn Pro Ala Pro Tyr Thr
   165 170 175
   Ile Ile Thr Phe Pro Phe Leu Phe Ala Val Met Phe Gly Asp Val Gly
   180 185 190
   His Gly Leu Leu Met Phe Leu Phe Ala Leu Ala Met Val Leu Ala Glu
   195 200 205
   Asn Arg Pro Ala Val Lys Ala Ala Gln Asn Glu Ile Trp Gln Thr Phe
   210 215 220
   Phe Arg Gly Arg Tyr Leu Leu Leu Leu Met Gly Leu Phe Ser Ile Tyr
   225 230 235 240
   Thr Gly Phe Ile Tyr Asn Glu Cys Phe Ser Arg Ala Thr Ser Ile Phe
   245 250 255
   Pro Ser Gly Trp Ser Val Ala Ala Met Ala Asn Gln Ser Gly Trp Ser
   260 265 270
   Asp Ala Phe Leu Ala Gln His Thr Met Leu Thr Leu Asp Pro Asn Val
   275 280 285
   Thr Gly Val Phe Leu Gly Pro Tyr Pro Phe Gly Ile Asp Pro Ile Trp
   290 295 300
   Ser Leu Ala Ala Asn His Leu Ser Phe Leu Asn Ser Phe Lys Met Lys
   305 310 315 320
   Met Ser Val Ile Leu Gly Val Val His Met Ala Phe Gly Val Val Leu
   325 330 335
   Gly Val Phe Asn His Val His Phe Gly Gln Arg His Arg Leu Leu Leu
   340 345 350
   Glu Thr Leu Pro Glu Leu Thr Phe Leu Leu Gly Leu Phe Gly Tyr Leu
   355 360 365
   Val Phe Leu Val Ile Tyr Lys Trp Leu Cys Val Trp Ala Ala Arg Ala
   370 375 380
   Ala Ser Ala Pro Ser Ile Leu Ile His Phe Ile Asn Met Phe Leu Phe
   385 390 395 400
   Ser His Ser Pro Ser Asn Arg Leu Leu Tyr Pro Arg Gln Glu Val Val
   405 410 415
   Gln Ala Thr Leu Val Val Leu Ala Leu Ala Met Val Pro Ile Leu Leu
   420 425 430
   Leu Gly Thr Pro Leu His Leu Leu His Arg His Arg Arg Arg Leu Arg
   435 440 445
   Arg Arg Pro Ala Asp Arg Gln Glu Glu Asn Lys Ala Gly Leu Leu Asp
   450 455 460
   Leu Pro Asp Ala Ser Val Asn Gly Trp Ser Ser Asp Glu Glu Lys Ala
   465 470 475 480
   Gly Gly Leu Asp Asp Glu Glu Glu Ala Glu Leu Val Pro Ser Glu Val
   485 490 495
   Leu Met His Gln Ala Ile His Thr Ile Glu Phe Cys Leu Gly Cys Val
   500 505 510
   Ser Asn Thr Ala Ser Tyr Leu Arg Leu Trp Ala Leu Ser Leu Ala His
   515 520 525
   Ala Gln Leu Ser Glu Val Leu Trp Ala Met Val Met Arg Ile Gly Leu
   530 535 540
   Gly Leu Gly Arg Glu Val Gly Val Ala Ala Val Val Leu Val Pro Ile
   545 550 555 560
   Phe Ala Ala Phe Ala Val Met Thr Val Ala Ile Leu Leu Val Met Glu
                   565                 570                 575
   Gly Leu Ser Ala Phe Leu His Ala Leu Arg Leu His Trp Val Glu Phe
               580                 585                 590
   Gln Asn Lys Phe Tyr Ser Gly Thr Gly Tyr Lys Leu Ser Pro Phe Thr
           595                 600                 605
   Phe Ala Ala Thr Asp Asp
       610